# EUROPEAN PATENT SPECIFICATION

(11) **EP 4 093 287 B1**
(45) Date of publication and mention of the grant of the patent: **28.10.2026**
(21) Application number: 21744927.1
(22) Date of filing: 22.01.2021
(51) Int. Cl.: A61B 5/28, A61B 5/349, A61N 1/04, G06N 3/08, G16H 10/60, A61B 5/00

(54) **METHOD, DEVICE AND SYSTEM FOR WIRELESS BIOPOTENTIAL MEASUREMENT**
VERFAHREN, VORRICHTUNG UND SYSTEM ZUR DRAHTLOSEN BIOPOTENTIALMESSUNG
PROCÉDÉ, DISPOSITIF ET SYSTÈME DE MESURE DE BIOPOTENTIEL SANS FIL

(30) Priority: 24.01.2020 NO 20200093
(43) Date of publication of application: 30.11.2022
(73) Proprietor: Appsens AS, 4790 Lillesand (NO)
(72) Inventor: FENSLI, Rune, 4818 Færvik (NO); JORTVEIT, Jarle, 4790 Lillesand (NO); YTTERDAHL, Tord, 4790 Lillesand (NO)
(74) Representative: Zacco Norway AS
(86) International application number: PCT/NO2021/050015
(87) International publication number: WO 2021/150122

(56) References cited:
- WO-A1-2017/108215
- WO-A2-2015/003015
- JP-A- 2018 094 412
- US-A1- 2015 223 716
- US-A1- 2016 120 433
- US-A1- 2017 340 206
- US-A1- 2019 133 486
- US-A1- 2020 357 517
- US-A1- 2020 357 519
- US-B1- 9 345 414
- US-B2- 10 052 022

## Description

### TECHNICAL FIELD

The present invention relates to a system, method and device for wireless biopotential measurement. More particularly, it relates to a sensor device for providing an Electrocardiogram (ECG) and arrhythmia analysis. The sensor device comprises two parts: a medical plaster patch, which may be disposable, and a reusable electronic device comprising communication means. The system is further comprising a communication unit, such as a smart phone, and a remote back-end service.

### BACKGROUND

Detection and diagnosis of heart problems and other medical concerns, often require the use of advanced detectors, and in the history of electrocardiogram (ECG) instruments spanning over more than 100 years, the sensor and instruments has been gradually improving and becoming more and more reliable and easy to use.

The most advanced instruments and sensors are invasive and requires surgical operation inserting sensors close to the heart, whilst in hospitals today the most common sensor devices rely on the non-invasive lead ECG standardized by the American Heart Association.

In US2017/0340206 it is provided a system and method for providing dynamic gain over non-noise electrocardiographic data with the aid of a digital computer.

In US2015/0223716 it is shown a heart activity sensor structure includes a flexible substrate being substantially non-conductive, at least two electrodes printed on one side of the flexible substrate and configured to be placed against a skin of a user in order to measure biomet-ric signals related to heart activity

The problems with these methods are that they are cumbersome to use, and require a lot of medical competence to execute.

Also, there is a need for extensive analysis of the collected data sets, requiring the use of many expert man-hours and thereby putting a high load on limited personnel resources.

There is a challenge to provide reliable detection and analysis of medical conditions without the use of expensive medical and hospital resources.

Even further challenges are evident in light of the fact that longer surveillance time, longer than minutes and up to days and weeks, may improve the ability to detect conditions with organs such as the heart, where a specific condition may only be occurring sporadically.

A problem to be solved by present invention can thus be defined as how to provide an easy to use ECG sensor device that can be used by non-professional medical resources, such as the patients/users themselves. And further that such a sensor device may be provided at a cost/use relationship that enables a wide distribution of such a product. Thus, a high number of people may afford and understand how to use the sensor device for early/safe detection of heart conditions before they appear to be critical and impossible to cure. The invention is further defined by the attached independent claims, and further embodiments of the invention are defined by the attached associated dependent claims.

### SUMMARY OF THE DISCLOSURE

The present disclosure is directed to an electrocardiogram sensor device and corresponding method of use as defined in the independent claims. Further aspects are defined in the dependent claims.

The internal of the electrocardiogram sensor device is designed to be electrically shielded from the surrounding and the shielding is coupled to the electrical potential of the person wearing the detector device , wherein a shielding electrode is connected with the conductive material of a second layer via apertures in the dielectric layers, ensuring electrical contact.

### BRIEF DESCRIPTION OF THE DRAWINGS

In order to facilitate understanding of the invention and explain how it may be worked in practice, non-limiting examples will be described with reference to the accompanying drawings, in which:
Fig. 1A-E Illustrates an embodiment of the sensor device
Fig. 2A-C Illustrates the layer structure of the disposable patch excl. power source and connector.
Fig. 2D Shows an exploded view of the disposable patch 2, the medical plaster 38 and the hydrogel fillings 37.
Fig. 2E Shows the disposable patch assembly illustrated in figure 2D seen from above and below.
Fig. 3A Shows a functional diagram of the sensor device and the shielding principles
Fig. 3B-D Shows one embodiment of the print-board and mounted electronic components of the reusable electronic device.
Fig. 3E Shows a functional diagram of the sensor device and the shielding principles with variable impedance resistors
Fig. 3F Shows a functional diagram of the sensor device and the shielding principles with separate noise-pick-up electrodes
Fig. 3G Principle diagram for noise influence
Fig. 4 Shows a data flow overview and module layout of one embodiment of a system according to present invention
Fig. 5 Describes a logic diagram of feature modules of a system according to the invention.
Fig. 6A Shows a typical hart activity diagram with the most commonly used reference letters and definitions.
Fig. 6B-I Shows variations in traditional ECG charts corresponding to actual arrhythmias to be detected by the sensor device.
Fig. 7A Shows a hart activity diagram from the analysis toolset of the present invention illustrating a detected Ventricular Extrasystole event
Fig. 7B Shows a hart activity diagram from the analysis toolset of the present invention illustrating a detected Atrial Fibrillation event
Fig. 7C Shows a section of a hart activity diagram from the analysis toolset of the present invention illustrating a detected Ventricular Extrasystole event
Fig. 7D Shows a hart activity diagram from the analysis toolset of the cellphone of the present invention illustrating a normal heartbeat
Fig. 8 A-B Data flow in use scenarios
Fig. 9 Example of results from training of Neural Network used in Al module training
Fig. 10A-E Balance and tradeoffs between False Negative and False Positive
Fig. 11A "sliding-window" principle
Fig. 11B Flow diagram for post processing algorithm
Fig. 12 Accuracy diagram for a ML ideal v.s. real world data
Fig. 13 2-step ML algorithm, unsupervised and supervised
Fig. 14A Importance configuration table
Fig 14B Focus region in beat

In the following description of various embodiments, reference will be made to the drawings, in which like reference numerals denote the same or corresponding elements. The drawings are not necessarily to scale. Instead, certain features may be shown exaggerated in scale or in a somewhat simplified or schematic manner, wherein certain conventional elements may have been left out in the interest of exemplifying the principles of the invention rather than cluttering the drawings with details that do not contribute to the understanding of these principles.

It should be noted that, unless otherwise stated, different features or elements may be combined with each other whether or not they have been described together as part of the same embodiment below. The combination of features or elements in the exemplary embodiments are done in order to facilitate understanding of the invention rather than limit its scope to a limited set of embodiments, and to the extent that alternative elements with substantially the same functionality are shown in respective embodiments, they are intended to be interchangeable. For the sake of brevity, no attempt has been made to disclose a complete description of all possible permutations of features.

Furthermore, those with skill in the art will understand that the invention may be practiced without many of the details included in this detailed description. Conversely, some well-known structures or functions may not be shown or described in detail, in order to avoid unnecessarily obscuring the relevant description of the various implementations. The terminology used in the description presented below is intended to be interpreted in its broadest reasonable manner, even though it is being used in conjunction with a detailed description of certain specific implementations of the invention.

When the electronic device described below is referred to as a reusable electronic device it shall be understood that even if a unique feature of the present invention is the reusability aspect of the electronic device, the invention may also be provided in an embodiment wherein the reusability aspect is not present.

When the patch described below is referred to as a disposable patch it shall be understood that even if a unique feature of the present invention is the disposability aspect of the patch, the invention may also be provided in an embodiment wherein the disposability aspect is not present.

The present invention provides several aspects that can be combined into a system for improved monitoring of ECG and arrhythmia analysis. The first aspect relates to the way data about ECG and arrhythmia conditions may be obtained, while additional aspects relate to aggregation of such data, processing of aggregated data, and analysis and sharing of processed data.

With the exception of structural details that are specifically developed for a particular type of sensor device, for example the disposable patch adapted for being attached to and worn by a person, male or female, the devices and methods described herein are adaptable for use with a wide range of equipment including not only the disposable patch for humans, but also electromagnetic sensor devices operating in for example electromagnetically noisy environments. For ease of understanding, the examples described herein will primarily describe embodiments where sensor devices are mounted to a person's chest for ECG and arrhythmia analysis, but the examples may be generalized to other types of equipment and purposes.

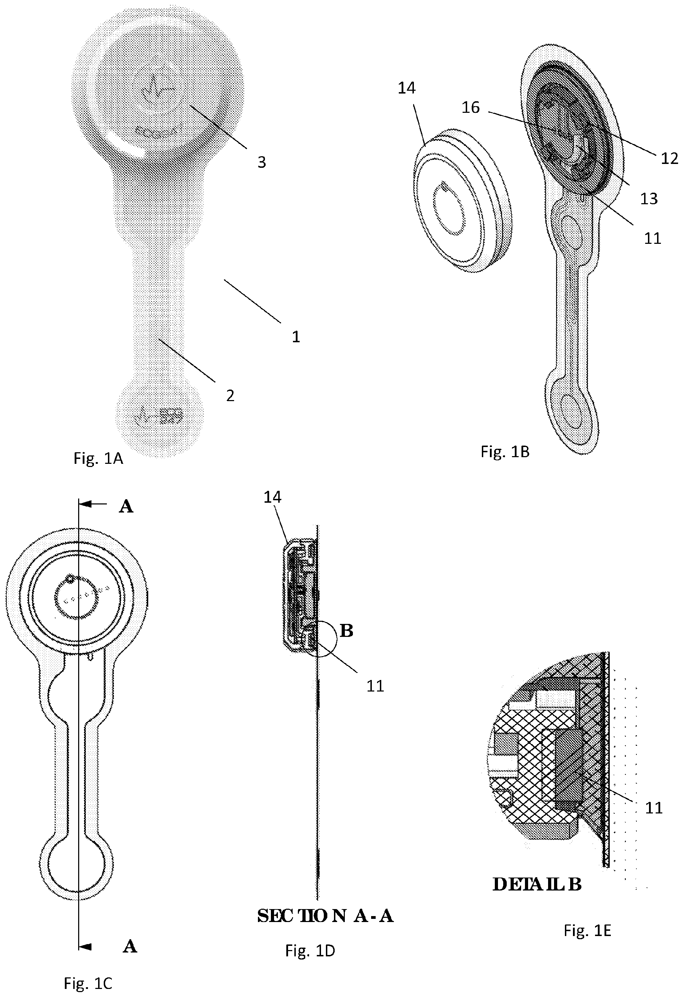

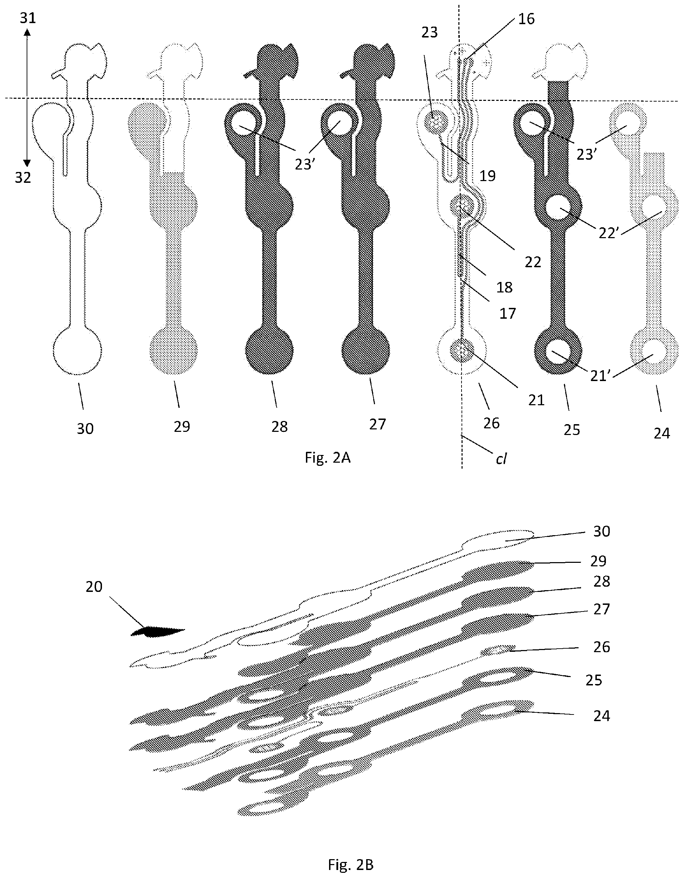

Reference is first made to FIG. 1A, which illustrates the sensor device 1 comprising a disposable patch 2 and a reusable electronic device 3. The disposable patch is provided as an assembly of a number of layers, the individual layers as exemplified in figure 2A and figure 2B, comprising at least two pick-up electrodes 21, 22 and a shielding electrode 23. The reusable electronic device 3 comprising at least a cover 14, data storage means, processing means, electrical connector for connecting to electrical wire connecor 16 of the disposable patch 2, and a wireless communication module for wireless communication with a further handheld/remote computer resource, such as for example a smart phone.

The reusable electronic device 3 comprise cover 14 attachment means for mounting and demounting the reusable electronic device 3 to and from the connector 12 of the disposable patch 2. The cover attachment means may be comprised of a bayonet type push and screw type design comprised in the cover 14 and a corresponding base portion of a connector 12 arranged on the disposable patch 2. A resilient seal 11 may be arranged between the cover 14 and the connector 12 for sealing the environment inside the reusable electronic device 3 once attached to the disposable patch 2. The reusable electronic device 3 may have a built-in switch/push button 33 in order for the user to turn the sensor on and start a Bluetooth pairing procedure with a remote device, for example a smart phone. A further LED indicator may be embedded to inform the user of the status of the sensor device.

Figure 2A illustrates exemplified layers comprised in the disposable patch 2 of the embodiment of present invention as seen in figure 1A. Figure 2B illustrates an exploded view of the different layers in the order they are preferably assembled. Other production/assembly order may also apply, depending on the various layer composition.

The disposable patch may comprise as illustrated in the figure a first protection layer 30 comprising protection for wear and tear, and may be composed of a PET material or other flexible durable material. Typically the cover may be printed with information and codes for correct usage.

A second layer of conductive material 29 for covering at least the area above the underlying layers wherein the pick-up electrodes 21, 22, the shielding electrode(s) 23, and the wiring are comprised. The shielding electrode 23 is connected with the conductive material of the second layer of conductive material 29 via apertures in the underlying dielectric ink 27, 28 arranged at the location directly above the shielding electrode(s) 23. Thus, electrical connection between the body through the shielding electrode to the second layer of conductive material 29 is ensured. The second layer of conductive material 29 may be composed of the material composition conductive silver ink (Ag/AgCl). Other conductive material may be used.

Layer three and layer four of a flexible dielectric ink 27, 28, is arranged under the conductive material layer. Both layers of dielectric ink 27, 28 has in this embodiment an aperture 23' for exposing the shielding electrode 23 upwards from the body whom the disposable patch 2 is intended to be attached to. The pick-up electrodes 21, 22 comprised in an underlying layer are shielded by dielectric material in the upward direction.

A first conductive layer 26 arranged underneath the layers of dielectric ink 27, 28 comprise two pick-up electrodes 21, 22 and a shielding electrode 23, further electric wiring 17, 18, 19, and a connector 16. The electric wiring 17, 18, 19 connects the pick-up electrodes 21, 22 and a shielding electrode 23 to the connector 16. The electric wiring 17, 18 from the pick-up electrodes 21, 22 is balanced to be of the same length and being arranged mostly parallel and close together, offering minimum exposure to ambient electromagnetic radiation. The wiring is further isolated from the environment by layers of dielectric shielding 25, 27, 28. The wiring and electrodes 21, 22, 23 of the first conductive layer 26 may be composed of the material composition conductive silver ink (Ag/AgCl). Other conductive material may be used. The pick-up electrodes 21, 22 are provided for measuring wirelessly the biopotential of the body to which the sensor device 1 is attached.

A further layer of dielectric ink 25 is designed to shield all the wiring tracks 17, 18, 19 of the first conductive layer 26 towards the body the disposable patch 2 is intended to be attached to. The further layer of dielectric ink 25 is arranged to cover an attachment layer 24. Both the attachment layer 24 and the further layer of dielectric ink 25 has through holes 21', 22', 23' arranged in positions corresponding to the two pick-up electrodes 21, 22 and the shielding electrode 23 of the layer of conductive material, the first conductive layer 26, for allowing direct contact between the body the disposable patch 2 is intended to be attached to and the pick-up electrodes 21, 22 and shielding electrode 23 . Present invention has been optimized for the two pick-up electrodes and the one shielding electrode, but it is within the scope of present invention to use other configurations, such as more pick-up electrodes and/or more shielding electrodes.

The attachment layer 24 is covered with an adhesive layer surface for fastening the patch to for example a medical plaster 38 for attachment to a human body. In an embodiment the medical plaster is a 3M 4076 SC plaster. In one embodiment the attachment layer is composed of material of type "3M 1567". Other adhesive material may be used. The medical plaster has corresponding holes with the pick-up electrodes 21, 22 and a shielding electrode 23, and the recesses underneath the pick-up electrodes 21, 22 and a shielding electrode 23 may be filled with an electrical leading material 37 such as for example hydrogel. The hydrogel fillings may improve the electrical contact between the pick-up and shielding electrodes 21, 22, 23 and the body.

The medical grade plaster 38 , such as "3M 4076 SC", will provide the function of gluing the sensor device to the user's skin.

All the layers may be composed of flexible materials, such that when the disposable patch 2 is worn by a person, the disposable patch 2 follows the movement of the body part it is attached to without too much discomfort for the person.

The various layers may cover the whole or portions of the disposable patch profile view. The top portion 31 of the disposable patch is intended for being threaded into the connector 12 and wrapped around a battery 13 that may be provided in the connector 12, such that the connector 16 is exposed towards the reusable electrical device 3 that is to be attached to the connector 12.

A further adhesive patch 20 is arranged on top of the upper part of the top portion 31 of the protection layer 30. The adhesive patch 20 is comprised in the layered structure to enable the top portion that is wrapped into the connector 12 and around the battery 13 to be attached (glued) to the battery for correct positioning of the connecting area 16 of the first conducting layer 26. The form of the top portion 31 of the disposable patch may have protrusions, for example a first protrusion 39 and a second protrusion 39' arranged to correspond to alignment elements of the base portion of the connector 12, in order to arrange the top portion 31 of the disposable patch at a known position in the connector 12. This is important for ensuring correct connection between the connector 16 and a corresponding connector 34 of the reusable electronic device 3.

The extent of the coverage area of the different layers 24, 25, 26, 27, 28, 29, 30, 20 varies according to the specific function the layer plays, and depends for example on the physical properties of the pick-up electrodes 21, 22 and a shielding electrode 23, the reusable electrical device 3, the connector 12 and other.

For example looking at the two layers of dielectric ink 27, 28 being provided to cover the whole area of the disposable patch 2 with the exception of the area of the underlying shielding electrode 23, is firstly to provide a good electrical contact between the shielding electrode 23 and the second layer of conductive material 29 arranged above. Secondly it provides a solid electrical shielding above the pick-up electrodes 21, 22, and all the wiring tracks 17, 18, 19 so these are electrically shielded towards the ambient environment.

The sensor 1 may have for the analogue signal part a bipolar amplifier construction based on principles described by Thakor and Webster (1980). However, the construction provides a wireless sensor system which is floating with respect to the electrical ground potential. By using a fully wireless solution, this will reduce the capacitance to ground for the amplifier system, and thus the capacitances between the body to which the sensor device is attached and ground versus between the sensor device amplifier and ground will almost be equal, and thereby having a reduced vulnerability to signal pickup from static noise and common mode 50 Hz disturbances. Traditionally, prior art ECG amplifiers will pick up common mode signal noise (50Hz disturbances) as this frequency is in the middle of the bandwidth for an ECG amplifier, and in order to reduce common mode disturbances a noise reduction method is used in such amplifiers, as the input signal is filtered and sent back to the body in anti-phase via a further electrode.

In the present invention construction, there is no use of such a further electrode. Instead, the electric wiring 17, 18 tracks provides electrical conduction between each of the two pick-up electrodes 21, 22 and the amplifier circuit, and are kept equal, or close to equal, in length and in close distance to each other and preferably symmetrical in design. The distance between the two pick-up electrodes 21, 22 may advantageously be less than 10 cm, or even less than 8 cm, or as little as less than 6 cm, and the two pick-up electrodes 21, 22 is arranged substantially in line or in line with a center line *cl*, and the two wiring track 17, 18 being substantially symmetrical in design and arranged close to each other and the center line *cl*. Such a layout will dramatically reduce the area (in cm²) that defines the area encompassing the two pick-up electrodes 21, 22 and the amplifier system, and this important design factor will reduce the pickup of signal noise. In traditional instrumentation for ECG apparatuses this corresponding area is defined by the ECG electrode wires, having a much larger areal. **In** addition, a shielding principle in the sensor disposable patch 2 design may be provided to prevent the ECG-amplifier system to pick-up static noise. This shielding principle is based on a "Faraday shield" where an infinite metal shield is placed above the two pick-up electrodes. In order to obtain electrical contact between this shield layer and a patient's skin, a third shielding electrode 23 is placed underneath the position of the attached electrical device 3. This shielding electrode 23 may in one design solution have no electrical connections to the ECG amplifier system, nor to the power source terminals, but can optionally in another design be electrically connected to battery - terminal in order to connect the floating electrical circuits to the "personal ground potential" at the user's skin. However, there will exist a capacitive coupling between this shield layer and the pick-up electrodes 21, 22 input terminals, which has to be taken into account when designing the instrumentational amplifier solution.

The shielding principle can be described as a passive noise protection.

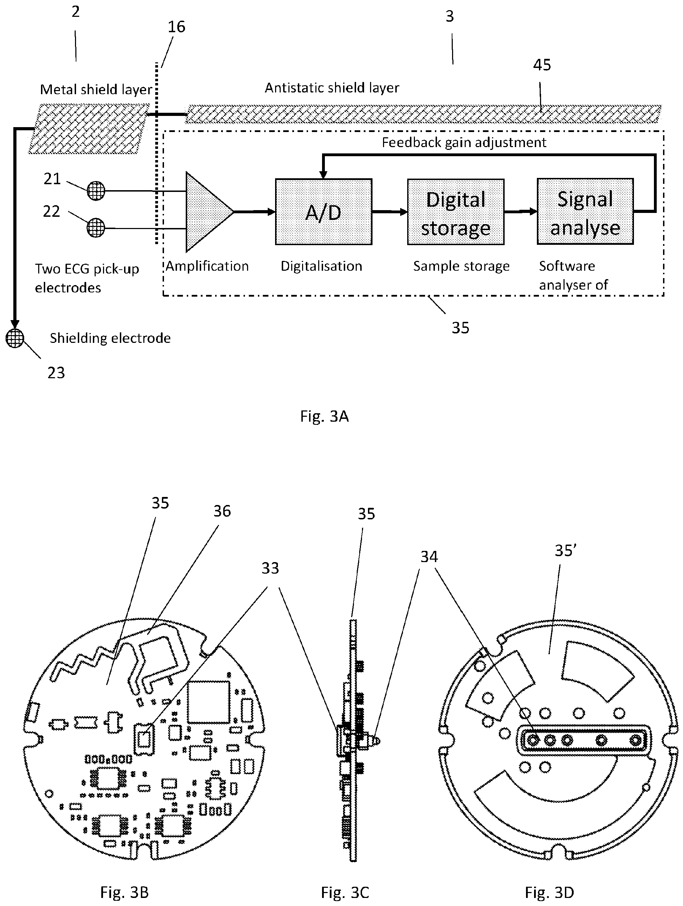

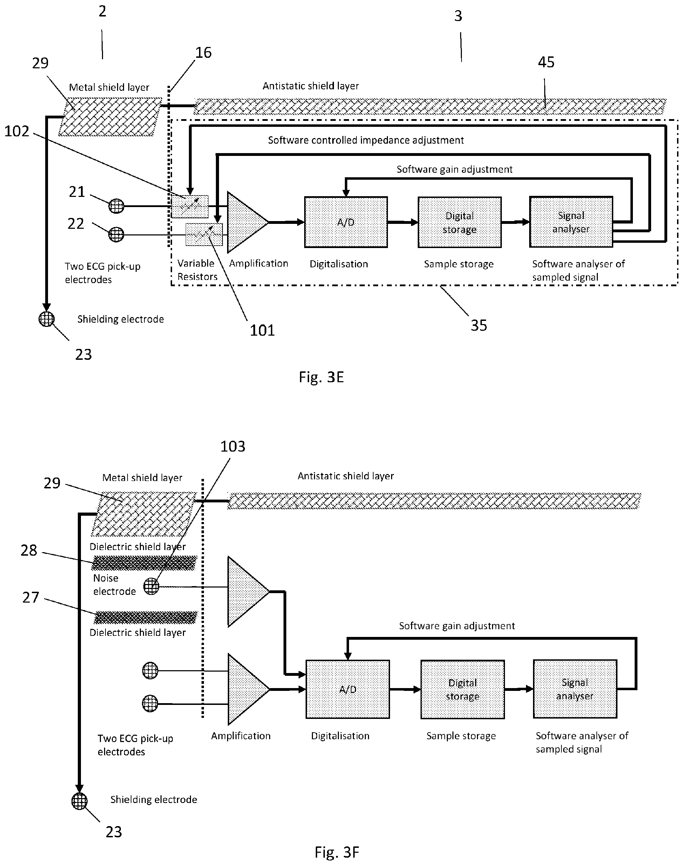

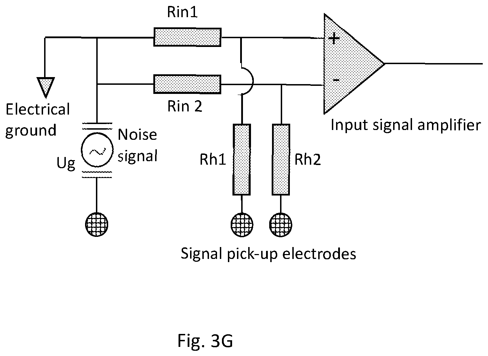

Signal noise may for a number of reasons be caused by imbalance between the electrode to skin impedance between the two pick-up electrodes 21 and 22. This imbalance in impedance may be detected by an impedance imbalance detector as principally describer by figure 3G below, and attenuated by introducing a variable impedance between the pick-up electrodes and the signal amplifier in the electrical device 3, wherein the variable impedance may be software controlled variable resistors 101, 102. If the total impedance between the two pick-up electrodes 21 and 22 is balanced, the influence of static noise may be substantially reduced. This principle may be described as an adaptive protection method, which may be individually optimized for the actual user. Figure 3E illustrates one such embodiment. It is within the inventive concept of present invention to comprise a variable impedance circuit controller being implemented in hardware at the input side of the signal amplifier.

A further noise cancelling method may be provided by arranging separate noise-pick-up electrodes 103 located between the layers of dielectric ink 27 and 28, and positioned longitudinally between the pick-up electrodes 21 and 22. The noise-pick-up electrodes will not be in direct contact with the user's skin. Thus, the signal measured by the noise-pick-up electrodes will not contain any ECG-signal and originates from noise disturbances only. By adding the signal measured by the noise-pick-up electrodes in anti-phase to the measured ECG-signal, also containing the noise, this will give a noise cancelling mechanism. Figure 3F illustrates one such embodiment.

Signal noise can be generated as an influence between the user's skin and the electronic amplifier circuits as shown for one possible embodiment in a simplified drawing in figure 3G. The noise signal generator will have a capacitive coupling to the user's body and also to the electronic print-board.

There are two resistors Rin1 and Rin2 which are mounted on the signal inputs on the print-board. At the same time there will be a resistance between the signal inputs at the print-board and the user's skin contact with the two electrodes, denoted as Rh1 and Rh2.

The influence from noise disturbances depends on the values of Rh1 and Rh2, which during normal use can vary between approximately 50 KOhm to 500 KOhm. Signal noise is in the following denoted as Ug.

Differential signal input to the signal amplifier can be calculated: $Ud = Ug * \left(Rh2*Rin2-Rh1*Rin1\right) / \left(\left(Rh1+Rin1\right)*\left(Rh2+Rin2\right)\right)$

A coefficient, K, is defined to describe how much the noise can be reduced and is calculated by: $K \left(Rh1, Rh2\right) = \left(Rh1+Rin1\right) * \left(Rh2+Rin2\right) / \left(Rh2*Rin2-Rh1*Rin1\right)$

The higher the coefficient K will be, the better noise suppression is achieved.

Rin1 and Rin2 will typically be equal in values, for example, but not restricted to, 5 MOhm.

In an embodiment wherein noise disturbance connections (resistance) Rh1 and Rh2 are almost equal, this will give a minimal noise influences in the ECG-signal measured. Also the lowest possible value of the resistance from the electrode skin contact, will give lowest noise influence.

If there are imbalances between the skin contact of the two pick-up electrodes 21, 22, this can be compensated using a software controlled variable resistor in the signal input terminals, as discussed for fig 3E above.

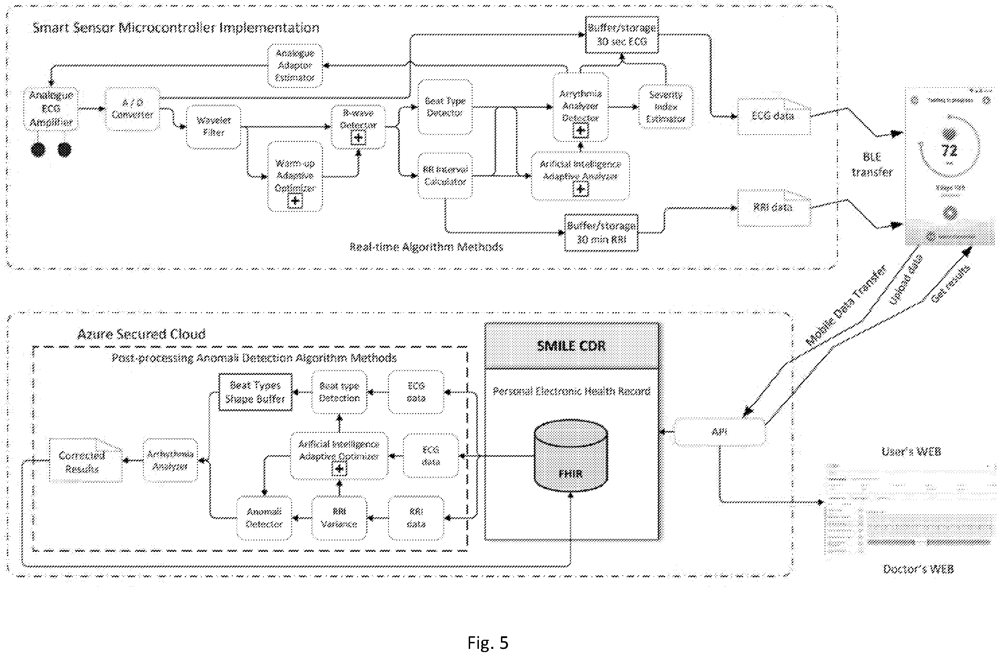

As seen in the functional diagram illustrated in figure 5, in one embodiment of the invention the software analysis controlling the variable resistor may be comprised in the Arrhythmia Analyzed Detector module. It shall be understood that this feature may be comprised in one or a combination of other modules within the sensor microcontroller.

Noise has many sources, signal distortions may in one instance be caused by the user rubbing against the surface of the sensor, or in a second instance be caused by the seatbelt of a car gently pressing against the user's chest. Such disturbances influences the detection of arrhythmia episodes in the ECG signals. In a further embodiment of present invention it is provided a method wherein the recorded signal is analyzed to find a pattern. Pattern changes or changes in the ECG signal rhythm may indicate an arrhythmia episode. Such patterns may be used for training the Al-system based on neural network models. If a different pattern arises, this may probably be caused due to signal distortions as mentioned above.

By analyzing and comparing a distorted signal to an anticipated pattern based on historical data from the user, detected for example in the seconds before the signal distortion occurred, wherein the distorted signal is detected based on its difference from the anticipated pattern, an anticipated noise pattern may be defined. Then subtracting the anticipated noise pattern from the actual measured pattern, an ECG pattern with reduced noise influences can be obtained. If impedance variances are sporadic and undefined, such variances may also be attenuated by the latter method.

Using an Al concept successfully for attenuating noise requires that the Al-system is well trained, and able to detect any real arrhythmia conditions, such that these arrhythmia conditions are not wrongly associated with noise.

The noise reduced ECG-signal is then passed on to an arrhythmia analyzer detector module for arrhythmia analyzes.

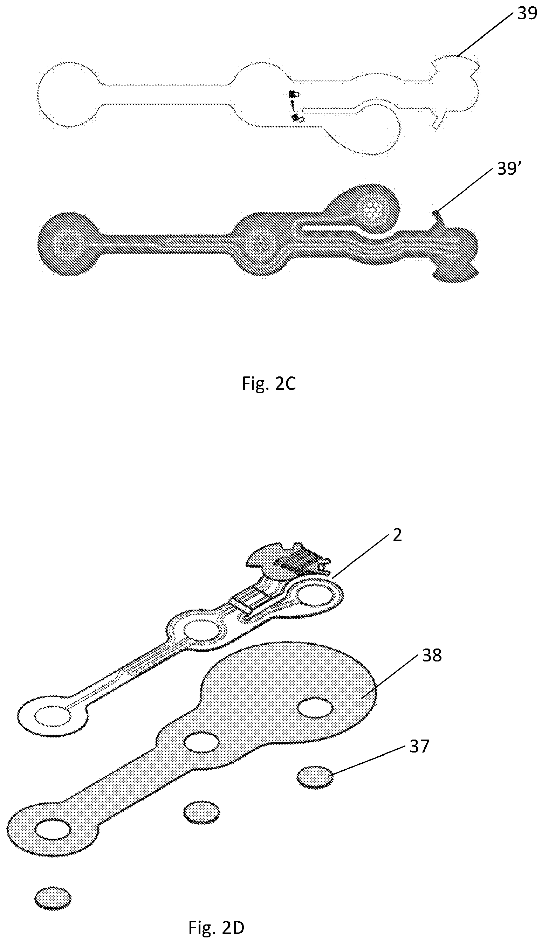

Figure 2C illustrate the disposable patch 2 from below and above when all the layers, minus the medical plaster, are arranged and fixed together. Informative ink is provided on the upper surface of the top protection layer 30 for guiding the user in the lock feature of how to fasten the reusable electrical device 3 to the connector 12.

Figure 2D shows an exploded view of the disposable patch 2, the medical plaster 38 and the hydrogel fillings 37.

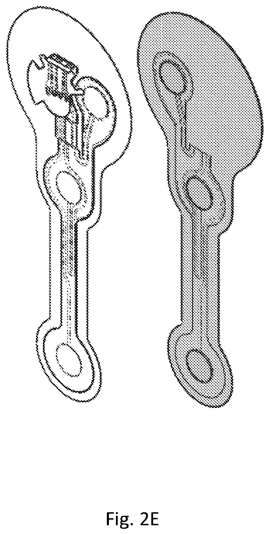

Figure 2E shows the disposable patch assembly illustrated in figure 2D from above and below.

Figure 3A illustrates a logic representation of the various parts of the disposable patch 2 and the reusable electrical device 3, wherein the connection between the two is illustrated by the dashed line representing the connector 16 of the disposable patch 2.

The reusable electrical device 3 comprise a metal shielding layer in order to avoid electrostatic disturbances influencing the low voltage ECG signals/biopotential measured by the pick-up electrodes 21, 22. In one advantageously embodiment this metal shielding layer is provided on the inner surface of the cover 14, wherein the cover 14 may be a plastic encapsulation that can also cause electrostatic disturbances. It is thus necessary, not only to protect the two ECG- pick-up electrodes 21, 22, but also to protect the signal tracks towards the reusable electronic device 3. This may be obtained in a way that the shielding electrode 23, which is arranged in the disposable patch 2 and positioned underneath the reusable electronic device 3, has an electrical connection in the second layer of conductive material 29 and via the connector 16 towards an electronic print-card 35 connector 34, wherein the print-card 35 is mounted inside the reusable electrical device 3. The electronic print-card 35 connector 34 may be in the form of POGO pins. On this print-card 35, along a portion of a circular surface, for example half of a circle circumference corresponding to the internal of the cover 14, an electrical contact is provided such that the said electrical conducting half of a circle circumference portion may provide a direct contact between the shielding electrode 23 and the inside of the cover 14 via the said second layer of conductive material 29. In one embodiment the inside of the cover 14 may be sprayed with semi-conducting material 45, which provides an electrostatic shielding also for the reusable electronic device 3, wherein the sensor 1 is grounded to an electrical potential of the person wearing the sensor 1. In a further embodiment, substantially all of the encapsulation may be made of an antistatic material with the result that all of the sensor device 1 is shielded towards unwanted electrostatic disturbances In this manner, the device establishes a shielding ground potential equal to the potential level of the body the disposable patch 2 attached to.

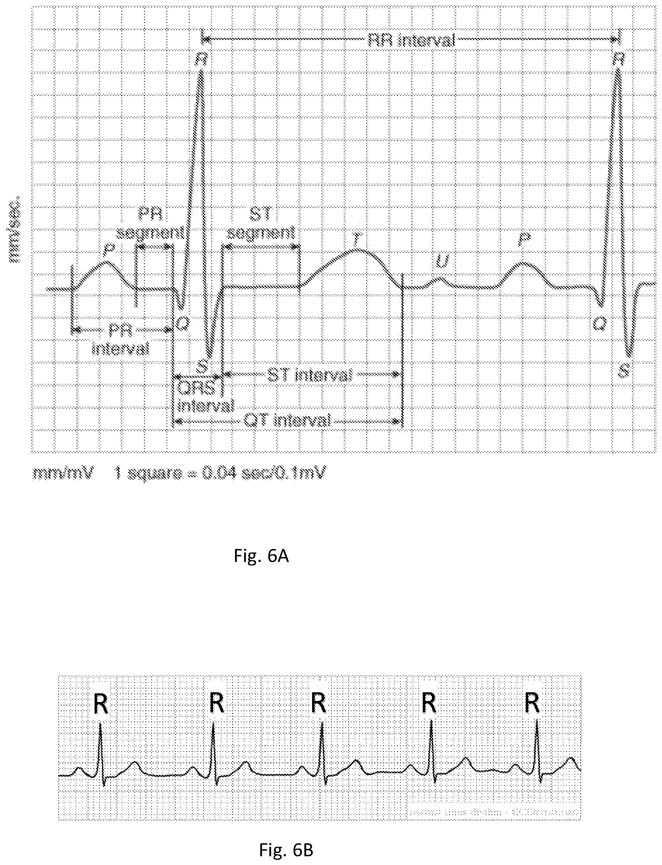

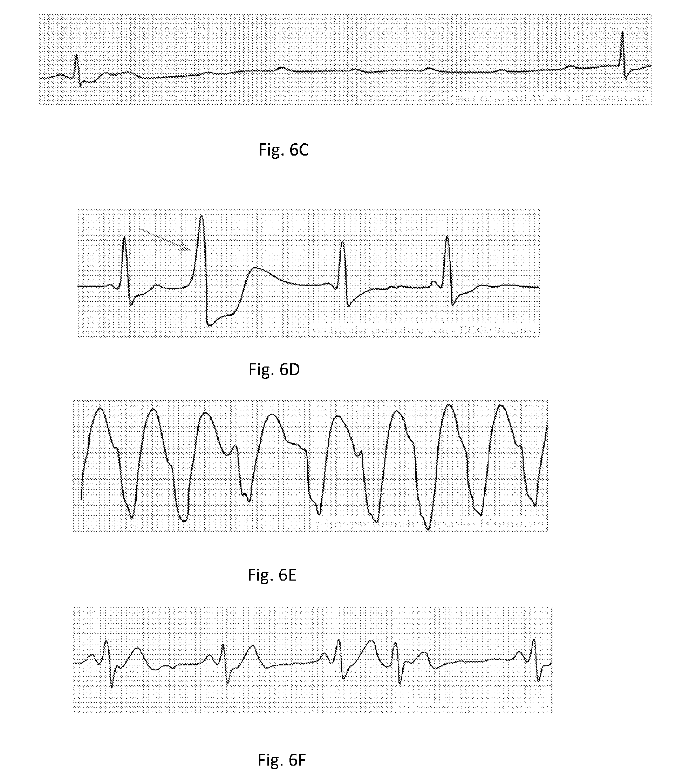

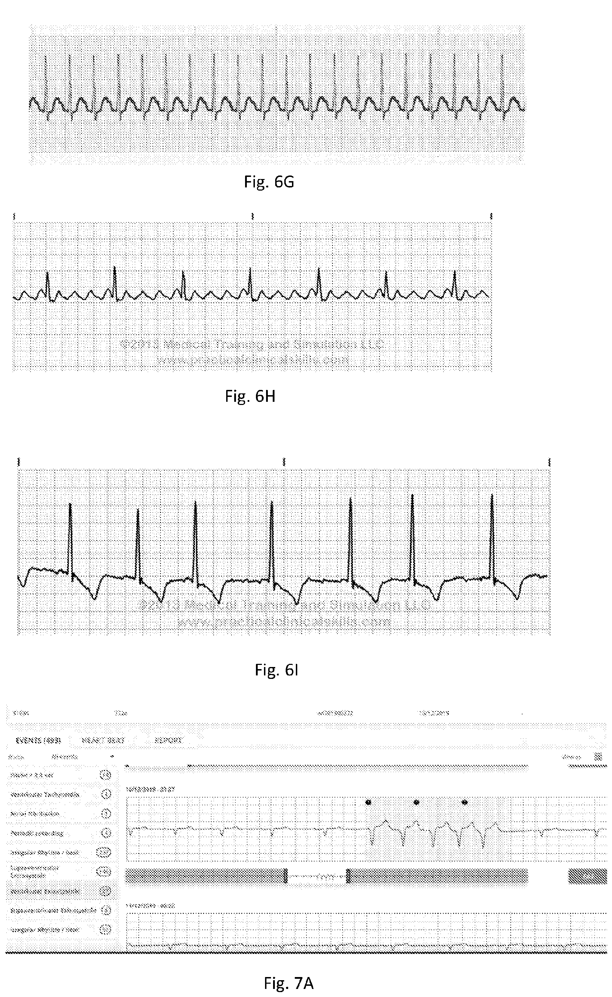

The impedance between the two pick-up electrodes 21 and 22 and the user's skin should be as low as possible and substantially equal in impedance, while the impedance between the two pick-up electrodes 21 and 22 and the input signal amplifier on the print-card 35 should be low and insignificant compared to the electrode-skin impedance, in order to balance any portions of signal disturbances such as from static electricity caused by cloths rubbing against the surface 14 of the reusable device. A typical sensor reading from such a disturbance sequence is shown in figure 6B of a Ventricular Extrasystole, which is initially detected as a false Ventricular Tachycardia event (red).

The print-card 35 may have an antenna 36 embedded. When covering the encapsulation with conducting material 45, it is important to avoid such covering in the area of the encapsulation where the antenna is located, in order to avoid attenuation of signals transmitted to and from the antenna 36. The antenna 36 may typically be implemented as a Bluetooth antenna. In alternative embodiments the antenna may be arranged external to the print-card, for example embedded in the cover 14 of the reusable electronic device 3 (not shown), or embedded in the protection layer 30 of the disposable patch 2 (not shown). An electrical connector (not shown) is provided between the antenna and a communication module comprised in the electronic circuits on the print-card 35.

The reusable electronic device 3 communication module provides support for wireless communication of sensor date, analyzed data and configuration data to/from a remote computing device. Remote computing device may be one or more of a smart phone, a computer, a network connected computer or cloud computing services.

The communication protocol in one embodiment makes use of Bluetooth communication between the sensor 1 and a portable communication unit 41. Depending on the portable communication unit 41 and/or the protocol and antenna providing communication with the sensor 1, a suitable antenna configuration may be selected to fit communication concepts of one of but not limited to: Wi-Fi, LTE, LoRa, NB-loT, GPS, Bluetooth, Zigbee, 802.15.4, and 5G.

It is within the inventive concept of present invention to establish communication directly between the sensor 1 and a back-end service 42.

The electric wiring 17, 18, 19, from the pick-up electrodes 21, 22 and the shielding electrode 23 is electrically connected to the reusable electronic device 3 via a connector 16 as illustrated by the dotted line in figure 3A. An amplifier device is electrically connected on its input side to the pick-up electrodes 21, 22.When the pick-up electrodes 21, 22 picks up an electrical signal from the body the disposable patch 2 is attached to, the signal is fed into and amplified by the amplifier device, and the amplified signal output from the amplifier device is then fed into an A/D converter. The signal is converted to a digital signal, and the digital signal is then stored and analyzed. A feedback portion of the analyzed signal may be fed into the A/D converter or the amplifier device for gain adjustment purposes.

Power source, such as a battery 13 is not shown in figure 3A, and may in principle be in either the disposable patch 2 assembly or reusable electrical device 3, although in a preferred embodiment as the illustrations show, the battery is arranged in the connector 12, and is part of the disposable elements of the present invention. This means that when the reusable electrical device 3 is reused on a new disposable patch 2, a new power source is also provided. In an alternative embodiment the power source may be a rechargeable battery arranged inside the encapsulation 14 of the reusable device 2 and optionally with a wireless charging arrangement.

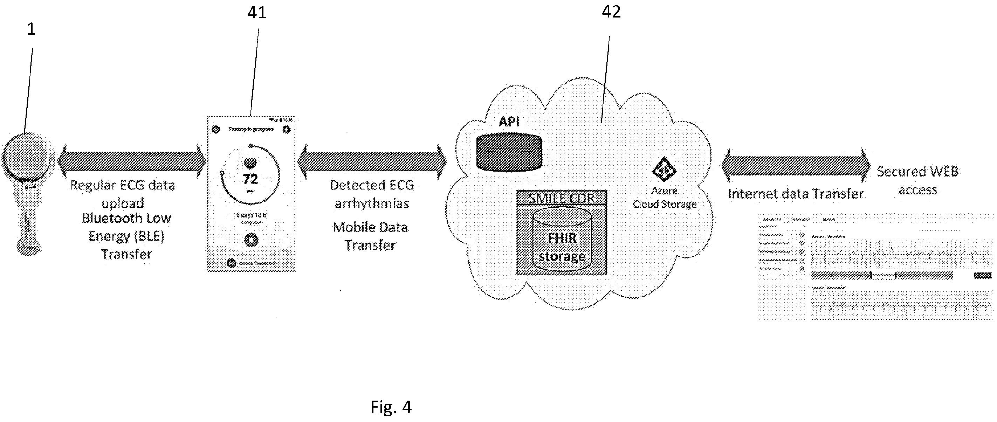

Figure 4 illustrates the data flow principles of an embodiment of a system implementation of the sensor device arranged to monitor a patient and being in communication connection with a portable communication unit 41, such as a smart phone, tablet or laptop, and a back end service 42 arranged at either a cloud computer or other computing resource running backend services.

The features that is implemented, for example as software routines running on an embedded microcontroller comprised in the sensor device 1, may record ECG-signals and analyze these data for arrhythmia episodes. The software routines may securely communicate with the portable communication unit via a dedicated communication protocol, such as for example a BLE protocol. The ECG-recordings and arrhythmia event codes representing analyzed arrhythmia episodes may be transferred from the sensor to a dedicated application, ECG-APP, running on the portable communication unit.

The ECG-APP may contain a graphical user Interface, ECG-APP GUI, for communicating with the user. The ECG-APP may further communicate data between the sensor device and the back-end services. The ECG-APP may communicate data via the ECG-APP GUI in real time directly from the sensor device, or the data may first be communicated from the sensor device to the back end services. The data may then be analyzed and user information may be prepared and transferred back to the ECG-APP where it may be communicated to the user via the ECG-APP GUI.

The ECG-APP in the portable communication unit 41 may connect also to other wireless sensors on the same user/patient in order to combine several signals within the analyzing and detecting system for remote monitoring.

One purpose of the present invention is to notify the user immediately if arrhythmia is detected, the notification may be sent as a push message to the ECG-APP.

The ECG -APP communication may comprise a two-way communication such that the portable communication unit 41 may be provided with options how to modify the sensor's behavior and how ECG-data is formatted and transmitted from the sensor.

During normal operation mode, the sensor may record ECG-signals and analyze for arrhythmia events. The RR-interval (time between two heart beats as discussed below) may continuously be recorded and automatically transferred to the back-end services for permanent storage (as a background service in the ECG-APP). In cases of detected arrhythmias, a predefined period, for example one minute, of ECG recording may be transferred to the back-end services as documentation of the detected event, and at the same time the user may be notified in the ECG-APP.

If the sensor arrhythmia detection algorithm detects an arrhythmia an event tag is allocated to the analysed data, defining the event, and sent to the ECG-APP.

Additionally, at regular predefined intervals, the sensor may record a predefined period, for example one minute, of ECG recording and automatically transfer the data-file to the back-end service. In a further embodiment it is provided for a system for continuously transmitting ECG-recordings as a remote monitoring system keeping a patient under surveillance.

In one embodiment of the ECG-APP a menu may be provided as a "Dashboard" wherein the Dashboard may provide an option to transfer instructions to the sensor device. Instructions such as: start continuous transmission and display of ECG-data, in order for the user to have a visual control of the ECG recordings, for example one or more of: a real-time ECG-graph that is displayed together with an indication of each heart beat detected, calculations of the actual heart rate, and if present arrhythmia conditions. By closing this "dashboard", the APP may transmit instructions to the sensor to return to normal operation mode.

A dedicated algorithm has been developed and implemented in the sensor's microcontroller, the arrhythmia detection algorithm, for real-time continuous analyzing recorded ECG-data for detection of possible arrhythmia events. There are defined several arrhythmia situations that can be detected, but perhaps the most important is the Atrial Fibrillation (AF) detection method.

The back-end services are provided with a similar or more advanced analyzing algorithm for analyzing the received sensor 1 data.

Both algorithm may advantageously be implemented according to Artificial Intelligence(Al)-principles, where the Al system is trained using ECG-data patterns from a data base of historical sensor data. This historical data base may be constructed from available arrhythmia databases, where the historical ECG-data patterns are analyzed for training of the Al system with deep learning algorithms and compared to the actual annotations for the arrhythmia database files. The method for training the Al-system may be based on some or all of, but not limited to, the following parameters within a heartbeat: a) R-R interval, b) Q-R amplitude, c) R-S amplitude, d) QRS-width, e) P-R interval, f) P-wave area, g) Deflection (positive or negative), h) Rhythm detected and i) Sudden change in rhythm, and compare the resulting detection of potential hart conditions as exemplified in table 2 below.

The sensor algorithm is trained and fine-tuned to detect: a) different types of heart-beats and b) different types of heart rhythms. It is of importance to avoid False Negative situations (the user has an arrhythmia not detected by the sensor algorithm); however, this can lead to an increased number of False Positive situations (the sensor incorrectly detects a situation to be an arrhythmia).The back-end service algorithm is trained and fine-tuned to analyze the detected arrhythmia episodes transferred to the back-end storage and to verify if this is a real arrhythmia or a False Positive situation. In cases where this is considered to be a False Positive, the corresponding annotations will be changed accordingly so when the user in the ECG-APP GUI 41 are shown the results, the number of False Positive situations are reduced.

The sensor device 1 of present invention has no accelerometer built-in, and detection of the user's activity level compared to arrhythmias may be based on detected changes in the heart rate and changes in the beat patterns.

Before the ECG-recordings are analyzed, a dedicated digital filter will remove low frequency components (often causing baseline wandering) and high frequency components (normally caused by muscle contractions and other artifact signals). This filter may be based on the frequency components in the signal as a typical discrete Wavelet transform filter.

At start-up of the sensor after successful Bluetooth pairing procedure between the sensor device and the mobile phone, a real-time clock in the sensor may advantageously be configured according to the local timecode used by the portable communication unit 41.

In principal, the algorithm may further contain a selection of the following types of functionalities, and is covered by the upper half of the functional diagram illustrated in figure 5:
a. At start-up the algorithm will begin a "warm-up" mode based on for example 30 sec. of recorded ECG signals, where the important issue is to analyze the user's ECG-signal for detection of heart beats. There is a search for a "Normal" or representative ECG heartbeat, and based on adaptive parameters estimated during this warm-up, important characteristics of the user's normal heartbeat is stored. These characteristics may also be stored in a back-end services database maintained for individual users, but also as data extracts for modeling a larger user group.
b. After detection of a heartbeat, the algorithm will re-analyze the 30 sec. ECG-data for detection of all heartbeats and calculate several adaptive parameters to be used by the algorithm in the continuous analyze. For example R-wave detection and RR interval (interval time from one R-wave to the next R-wave) and parameters as defined in this document.
c. If the signal quality is found sufficient, and a "Normal" heartbeat is detected, the algorithm may push information to the user's APP in order to start up the planned investigation period. If the signal quality is unsatisfactory or a "Normal" heartbeat is not detected, the user may be informed in the APP, and a new warm-up period may be initiated.
d. When starting a new investigation period, 1-minute of ECG is recorded and transferred to the back-end service acting as a "reference ECG".
e. When starting in normal operation mode, the algorithm, will continuously analyze the recorded ECG-data sample by sample. In one embodiment of present invention a sampling frequency of 256Hz is used, which gives an interval between each sample of 3,9 msec. Other sampling frequencies may be provided. During such a timeslot, all necessary calculations needs to be executed for the algorithm to analyze the ECG-data in real-time. In one embodiment of the present invention during this operating mode, there may not be any communication between the algorithm and the APP, nor to the back-end services, and no parameters are exchanged to customize the algorithm functionalities or characteristics. Thus, the sensor is self-contained in the developed algorithm to analyze ECG-signals and detect arrhythmia events after the warm-up period which calculates the necessary personal adaptive parameters.
f. When an arrhythmia event is detected, the sensor has in present example a storage content comprising for example 30 sec. history of ECG-recorded data and will continue to detect ECG-data for example for another 30 sec. in order to collect in total 1-minute ECG data as a documentation of the detected event. This data-file may be automatically transmitted to the ECG-APP which may forward this file to the back-end services without any user interaction.
g. Detection of Atrial Fibrillation, AF, is normally based on calculations of variance in the RR-interval. In order to accomplish such real-time calculations in the sensor device processor, this requires several computational operations, which can be difficult to achieve within the defined processing time-interval. At the same time there are constraints on the power consumption that has to be taken into account. Therefore, the present invention provides a more simplified approach based on calculations of an average RR-interval for normal beats, wherein a history of 10 normal beats is weighed such that the last beats has more weight. Further calculation of the power of the deviation for an RR-interval exceeding a predefined normal variation is investigated. If the calculated power of the deviation exceeds a defined limit for 10 succeeding heartbeats, then the analyze will detect this episode as a possible AF-event. In addition, the algorithm will try to calculate the presence of a P-wave, which is supposed to be located at a defined time-interval prior to the detected R-wave peak. In an AF-rhythm, the P-wave will appear at a high frequency and independent of the R-wave; therefore, it is anticipated that as an average for 10 beats in an AF-episode, the averaged P-wave area will be approximately close to 0 (as random noise will be calculated instead of a normal P-wave) and at the same time the time interval between the detected P-wave and the R-wave will exceed predefined limits based on clinical experience. By comparing this calculation of the P-wave area and time of occurrence to the warm-up calculated normal beat with a defined P-wave, it is possible to detect an episode without distinct P-wave, also indicating an AF-episode. If both those criteria are fulfilled, the algorithm will give a higher calculated probability for a detected AF-episode. In addition, Al-methods as defined in this document may be used in order to analyze possible AF-episodes for faster verifications.
h. File format for the detected for example 1-minute ECG-recorded data may be stored according to the standard ISO 22077 Medical Waveform Format. The same standard may be used for storing RR-intervals and arrhythmia events. Alternatively, RR-intervals may be stored in a csv-file or other format.
i. Time coding of all detected arrhythmia events may be according to a real-time clock in the sensor, as there can be a slight time-delay between the sensor and the ECG-APP using the portable communication unit 41, for example smart phone, clock.
j. User notification is comprised as a useful feature in present invention, and as the sensor is analyzing the ECG-signals in real-time or close to real time, the user may in the ECG-APP be presented with immediate information of arrhythmias detected. The ECG-APP may then also confirm a user's experiences of un-normal heart beats.
k. The sensor has a function where the user either by pressing on the surface of the sensor device or by pressing a button in the GUI-function in the APP, initiate a manual 1-minute ECG recording in cases where the user have a feeling on un-normal heart beats.
l. The sensor algorithm may comprise a signal quality analyze watchdog function with the aim of detecting situations where the sensor will lose skin contact or have poor skin contact causing a reduced ECG-signal quality. In such situations, the user may be warned in the APP, and instructed to take a visual control of the sensor and how this is fastened to the skin.
m. In the ECG-APP, the user may have full control of the on-going investigation, with information of recording time and remaining time for the scheduled investigation procedure (in number of days and hours), and may have a real-time display of the ECG recordings and arrhythmia episodes detected.
n. After finishing the scheduled investigation, the user may have to terminate the continuous ECG-recording and analyzing by for example sending shut-down commands to the sensor via the ECG-APP. This will force the sensor to stop functioning, and the APP can be closed.

The lower half diagram in figure 5 comprise some of the available back-end services and functional blocks.

The user may create a private account on a secured cloud service in the back-end server of present system invention at start-up. All registered ECG data, RR-intervals and detected arrhythmia episodes may then be stored in the back-end server. A dedicated investigation report may automatically be created by the back-end services.

This report may be downloaded as an encrypted password-protected PDF-file to the portable communication unit 41ECG-APP or sent to the user's email account.

The user may be provided with a secured access to a web-service wherein all detected arrhythmia episodes and the ECG-recordings belonging to the sensed data may reside and can be retrieved from. A general description of the detected arrhythmias will be available to the user.

The back-end services or the ECG-APP may provide the user with an option of getting access to an on-line cardiology specialist for evaluation of the automatic detected arrhythmia findings.

The user may also give a named local doctor (GP) or another person or services based on automated data processing, access rights to his/her stored data for third party evaluation. This may be provided by generating a secured one-time-code in the ECG-APP GUI, and this code can be given to the actual person or service being granted access. When this entity tries to access the actual stored data, a confirmation code may be sent to the user's portable communication unit 41 as a push-message to be confirmed or acknowledged.

The back-end services or ECG-APP GUI may give the user an overview of all consents given to other persons or services for access to the stored data, and the user may at any time withdraw this consent; thus stopping a person or service from having access to the stored data.

There are possibilities for electronic integration between the secured cloud service and the actual doctor's Electronic Health Record system based on the implemented FHIR server structure.

Further a generated report may be structured according to international specifications, such as for example given by HL7 FHIR Clinical Diagnostic Report, which can be electronically transferred to an actual health care service.

It is further provided a method for adaptive ECG analyze functions.

As the recorded ECG signals can vary between individuals, and also can change in shape during use because of body position and physical activities, an adaptive function is implemented in order to reliably detect a real heart-beat and to distinguish this from artefact disturbances. The first part of the adaptive process starts at the "Warm-up" phase, where significant signal components are analyzed for 30 seconds of ECG recordings as discussed above. By repeating the analyzing sequence, the typical shape of a dominant heart-beat may be identified. Parameters used for identification includes shape, deflection direction, deflection height, intervals between beats, wherein a normalization process may be used to define a typical beat shape. If the actual beat parameters are in accordance with parameters for a Normal beat-type, the actual shape will be stored as the identified Normal beat.

There might be cases where the user/patient can have frequent Ventricular types of beats, and in such cases there will be identified two different type of significant beats. Based on comparison with beat parameters corresponding to a Normal heart-beat, it is possible to distinguish which one of the detected two dominant beats is to be defined at the Normal beat shape. This adaptive process will be repeated for each Periodic recording of for example1 minute ECG, and used for updating the defined Normal beat shape. During analyzing ECG recordings, this normalized shape for a defined Normal beat may be used for correct detection of beats, and will make it possible to avoid incorrect detections of artefact disturbances.

Al-methods in the back-end services may be provided for post-processing and analysis of the transferred data and the detected arrhythmia episodes. The algorithm can be based on Al-principles where defined beat patterns are compared to a learning base. This learning base can be trained from available arrhythmia databases, where the defined beat patterns are analyzed for training of the Al system with deep learning algorithms and compared to the actual annotations for the arrhythmia database files. The method for training the Al-system may be based on detection of the following parameters within a heartbeat: a) R-R interval, b) Q-R amplitude, c) R-S amplitude, d) QRS-width, , e) P-R interval, f) P-wave area, g) Deflection (positive or negative), h) Rhythm detected and i) Sudden change in rhythm.

The present invention aims to detect and to avoid false positive investigations. One obvious example situation which generates false positives is if the user suddenly intensifies muscle activates such as starting to run. Thus, there will be generated a fair portion of false positive findings. The post-processing method may reduce the number of false positive findings. Arrhythmia event detections sent from the sensor device and the ECG-APP to the back-end services will in the storage in this setting be defined as Observations with Interpretations as "Preliminary".

Using Al and deep learning methods, these events may be customized for each individual user or stereotypes of users. The learning method may make use of arrhythmias detected by all users/sensor devices and wherein an evaluation by a cardiologist may be provided as feedback correction to the system.

All communication protocols may advantageously be secured by state of the art secure communication protocols.

Typically, all data received from the sensor device by the portable communication unit 41 will be transferred to the back end services. The ECG-APP features may comprise:
- communicate data between sensor device and back-end service
- communicate warnings and alarms between the device and back end service
- communicate configuration data from the back end service to the sensor device
- display instructions to the user on how to start-up a new investigation
- display status messages on an ongoing investigation
- display real-time ECG-signals streamed from the sensor device
- display information from the back end service, based on warnings and/or alarms
- update the sensor firmware to the latest available version
- Personalized configuration data

The back end services may be provided as a cloud service, such as for example a Microsoft Azure cloud service, where the clinical data repository SMILE CDR or equivalent may be implemented. As a part of the SMILE CDR ( clinical data repository) an HAPI (Health Level Seven API) FHIR (Fast Healthcare Interoperability Resources) server may also implemented.

In the latter case; all communication with the back end services may be routed through a dedicated API middle layer, where a set of defined search procedures are provided as a service for any specific user. Security protocol may define which search procedures and services is available at any set time and for any specific user.

It is within the inventive concept of the present invention foreseen that the portable communication unit 41 may comprise all the features and services provided by the back-end server/services discussed in this document. Thus in one embodiment the complete system is provided without a physical presence of the back-end service as a remote entity. IN such an embodiment the portable communication unit 41 will act as a "cloud"-resource.

ECG-recorded data and event annotations may be stored in a standard format in the back-end services, such as in the Medical Waveform Format in accordance to ISO 22077-3. All information transferred from the ECG-APP to be stored in for example the SMLIE CDR may be coded according to FHIR specifications. All arrhythmia events may be coded according to the standard Systematized Nomenclature of Medicine-Clinical Terms, SNOMED CT, ontologies.

A WEB solution providing services from the back-end services may be provided to give the user an overview of the actual investigation and arrhythmia findings associated with the analysis of the data logged, analyzed in, and transmitted from the sensor device. Detected arrhythmia episodes are grouped according to a defined dedicated Severity Index.

Web access to the back end services may be granted based on an access policy which may differentiate access level according to needs/requirements.

The system may further incorporate at some or all stages a two-factor authentication for access to the data and analysis. When data is to be shared, this shared access to stored data may be based upon the data owner's consent. At any time, the owner may in a web-interface and in the ECG-APP GU have an overview of consents given and can at any time recall a given consent. Information security solutions may be based on recommendations from national authorities. Information security may also be implemented according to requirements from the GDPR (General Data Protection Regulation).

The ECG signal sensed by the pick-up electrodes 21, 22 may be analyzed a first time in the reusable electronic device 3, and in figure 5 the upper half of the diagram denoted Smart Sensor Microcontroller outlies the various logical analyzing modules that may be comprised in an embodiment of the present invention. It is further appropriate to discuss some of the additional scenarios that is worthwhile for a sensor device 1 to pick up and forward to the user or the back-end services. Some of these are explained in relation to figure 6A -I.

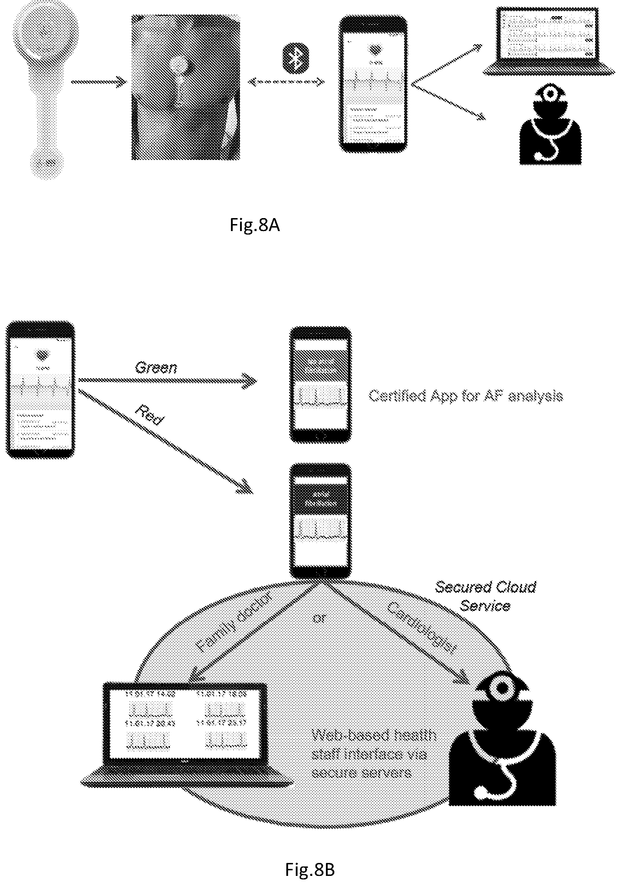

The pickup electrodes of the present invention is when attached to a human body attached longitudinal to each other, normally just above the solar plexus region as seen in the example of figure 8A. The longitudinal position of the pickup electrodes of present invention is perpendicular to the orientation of a traditional ECG sensor arrangement. The measured biopotential thus has an opposite polarization compared to the traditional measurement of an ECG signal plot. Figure 7A-D illustrates a selection of ECG plots o present invention and is illustrated below. It can be seen that the P curve is negative, the Q positive, the R negative, the S positive, and T negative, exactly the opposite of the rhythm as described for example in figure 6A which is taken from a traditional ECG detection instrument.

First a brief discussion of a normal ECG signal detected from a healthy beating hart is shown in figure 6A. An ECG curve contains waves P, Q, R, S, T, and sometimes U. For description of ECG are very important intervals and segments between waves. Every ECG description normally start with description of heart rhythm (regularly or irregularly, sinus or non-sinus rhythm) and frequency.

Figure 6B - I shows the following:
- 6B - normal sinus rhythm (SR)
- 6C - RR Interval > 2800m
- 6D -Ventricular extrasystole (VES) A VES is an ectopic beat that originates from the ventricles. The QRS width is at least > 120 ms. The VES is usually followed by a compensatory pause.
- 6E - Ventricular tachycardia (VT). A sequence of three or more ventricular beats with frequency >100 bpm
- 6F - Supraventricular extrasystole (SVES). Premature atrial complexes origin from an ectopic pacing region in the atria. The result is a premature p-wave with often a different morphology from the preceding ones and a premature narrow QRS complex (<120 ms).
- 6G - Supraventricular tachycardia (SVT). SVT is a rapid onset regular tachycardia with heart rate >150 bpm and QRS width <120 ms
- 6H - Atrial Flutter. During atrial flutter the atria contract typically at around 300 bpm, which results in a fast sequence of p-waves in a saw tooth pattern on the ECG. For most AV-nodes this is way too fast to be able to conduct the signal to the ventricles, so typically there is a 2:1, 3:1 or 4:1 block, resulting in a ventricular frequency of 150, 100 or 75 bpm respectively.
- 6I - Atrial fibrillation. During atrial fibrillation the atria show chaotic depolarization with multiple foci. At the AV node 'every now and then' a beat is conducted to the ventricles, resulting in an irregular ventricular rate (irregular RR interval).

### Example of Arithmetic calculations of variance:

Calculate mean RR-interval for the last 10 beats (RR-intervals) $GM = Sum \left(RRn+RRn-1+RRn-2……..\right) / 10$

Calculate the difference between the mean:
(RRn-GM), (RRn-1-GM), (RRn-2 -GM) ....

Sum the square of the differences: $DSUM = \left[\left(RRn-GM\right)2+\left(RRn-1-GM\right)2+\left(RRn-2-GM\right)2….\right] / 10$

Calculate mean RR-interval for the last 10 beats (RR-intervals) $GM = Sum \left(RRn+RRn-1+RRn-2……..\right) / 10$

A Simplified calculation model is presented in according to present invention: Based on calculation of a variance between two beats and array of 10 beats variance
Squared difference between 2 RR-intervals: $Vn = \left(RRn-RRn-1\right) * \left(RRn-RRn-1\right)$

For each beat, calculate the new Variance Vn
Values for V above an estimated limit is defined as an AF- un-normal variance
Define an AF-Array for 10 beats
   ∘ Set AFn = 1 if calculated Vn > estimated limit else AFn = 0
   ∘ IF the sum of AF-Array > 9 then we have a positive and AF-alarm is set to true, then Rhythm = AF-rhythm
   ∘ IF the sum of AF-Array <=9 then AF-alarm is set to false

If AF-alarm =True, then continue to test for the sum of AF-array.
∘ If the sum of AF-Array returns to < 5 then reset Rhythm back to Normal Sinus rhythm

The following severity code scheme is postulated and presented:

Figure 7A shows a hart activity diagram from the analysis toolset of the present invention illustrating a detected Ventricular Extrasystole event. The sensor device has first detected an Arrhythmia event and the defined sequence of registered data has been sent to the back-end server for further analysis, Thus in this example it can be seen that the arrhythmia sequence was not considers serious since it recovered fairly quickly, for example within 5 heart beats. "GREEN" code highlights a non serious event.

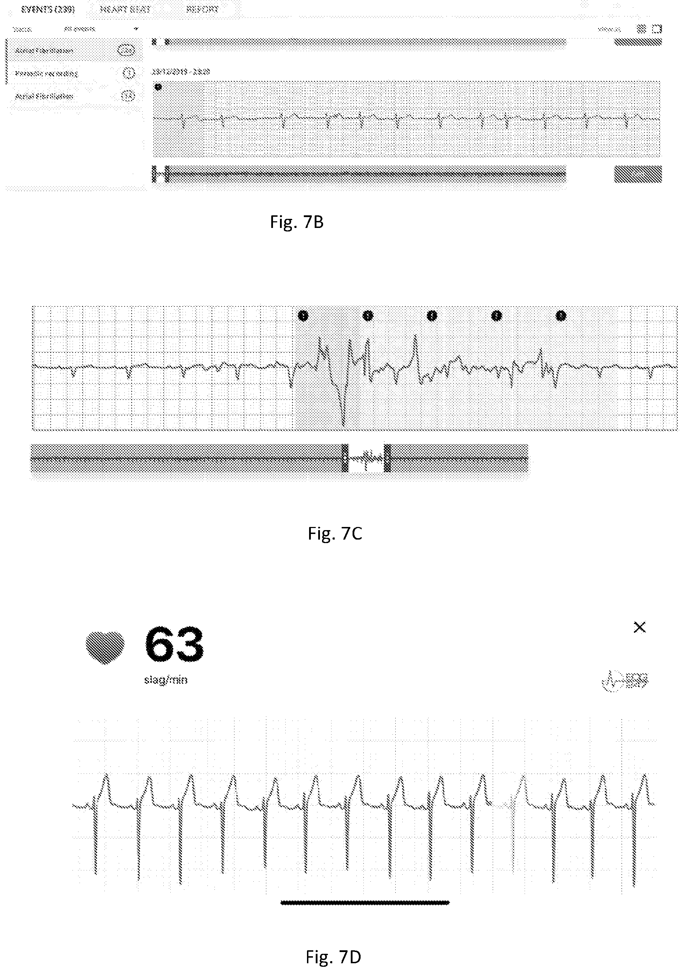

Fig. 7B shows a hart activity diagram from the analysis toolset of the present invention illustrating a detected Atrial Fibrillation event. Since the arrhythmia event is detected for more than for example 5 heart beats, the sequence is coded "RED"-

Fig. 7C shows a section of a hart activity diagram from the analysis toolset of the present invention illustrating a detected Ventricular Extrasystole event, first detected as RED, but when the heart beat returns to normal within the preset number of heart beats, for example 5, the event is considered to be normal, "GREEN". The event is probably caused by static noise. or a variable impedance has been detected and the software controlled variable resistors 101, 102 has adjusted the attenuation in the amplifier to remediate the variable impedance situation.

Fig. 7D shows a hart activity diagram from the analysis toolset of the cellphone of the present invention illustrating a normal heartbeat

**Table 1: severity code**

| **Severity Code** | **Colour** | **Interpretation** | **Comments** |
|---|---|---|---|
| 01 | GREEN | Normal | |
| 02 | YELLOW | High | proprietary coding schema. |
| 03 | ORANGE | Significantly high | |
| 04 | RED | Critical high | |

**Table 2: Severity code scheme**

| **Name** | **RED 04** | **ORANGE 03** | **YELLOW 02** | **GREEN 01** |
|---|---|---|---|---|
| Ventricular Tachycardia | ≥30 sec | | < 30 sec and > 4 beats | |
| Pause > 2,8 sec | X | | | |
| Heart Rate < 30 bpm | ≥30 sec | | | < 30 sec |
| Supraventricular tachycardia | ≥30 sec | | < 30 sec and > 15 sec | < 15 sec |
| Atrial Fibrillation | | ≥30 sec | < 30 sec and > 15 sec | < 15 sec |
| Atrial Flutter | | ≥30 sec | < 30 sec and > 15 sec | < 15 sec |
| Heart Rate > 180 bpm | | | ≥ 5 min | < 5 min |
| Irregular Rhythm/beat | | | ≥30 sec | < 30 sec |
| Ventricular Extrasystole | | | | X |
| Supraventricular Extrasystole | | | | X |
| Normal Sinus Rhythm | | | | X |
| Normal beat | | | | X |
| Manually recording | | | | X |
| Periodic recording | | | | X |
| Unsatisfactory signal quality | | | | X |

Thus in one use scenario of the present invention the following may be the case:
The present invention may use the coding schema for ECG-files and detected arrhythmias, wherein the sensor will have incorporated algorithms for real-time analyzing the ECG-signals for detection of heart beats (R-wave) and arrhythmias (see above).

The coding schema for the communication of data between the sensor 1 and the ECG-APP is typically customized and communicated via the BLE interface. After a stream of ECG data is transferred from the sensor device 1 to the ECG-APP, the ECG-APP will create a file, and the coding schema will be used both for defining the file-name and for the coding of MFER-files to be transferred and stored in the back-end service.

If a more serious arrhythmia is detected during the recording period, the severity event placed in the end of the file, and used for file-naming will always ensure that the most severe arrhythmia type is denoted.

A middle-layer service may be used for mapping the actual codes used by the sensor device 1 and the ECG-APP for correct input and storage in for example the SMILE CDR and FHIR-services, including the use of actual Snomed codes.

The principles for sensor algorithm detection and coding may be organized in separate activities.

### Beat detection :

The algorithm implemented in the senor device 1 may continuously analyze every ECG-signal sample, in order to detect a heartbeat. When a heartbeat is reliably detected according to the implemented algorithm principles, this will be stored as a beat-event together with the timing, in order to calculate the actual RR-interval.

### Rhythm detection:

The algorithm will further analyze the detected heartbeats in order to detect arrhythmias according to the implemented algorithm principles. When an arrhythmia is encountered, this will be stored with a rhythm-event together with the timing of occurrence in order to calculate the duration.

### Rhythm Severity Index:

The algorithm will calculate the duration of an arrhythmia episode detected and will define the actual arrhythmia with a Severity Index, as an information-code. This index will be important for how the actual arrhythmia detected will be displayed both to the user and to a doctor or health-care professionals.

### ECG files and content:

The sensor device 1 will have internal storage of for example 30 sec. historical ECG-samples prior to detection of an arrhythmia. In case of a detected arrhythmia, the sensor device 1 will start streaming ECG-samples to the ECG-APP together with the actual event-codes. Normally the length of an ECG-file will be 1-minute, but if the arrhythmia has a duration of more than for example 30 sec., this time can be prolonged for up to several minutes, for example 4 minutes, of recordings. When the actual arrhythmia has stopped and the sensor device 1 detects a normal rhythm, the recording time will continue for another time period, for example 30 sec. After the actual arrhythmia event has stopped, or after each prolonged period of streamed ECG-data, the ECG-APP will put together received data from the sensor device 1 as an MFER-file and transfer this file to the back-end service. The file name may further contain important parameters defining both the user and detected arrhythmia as described above.

For coding of ECG recordings, present invention may use the specifications of Medical Waveform Format (MFER) as defined by well-known standards. In principle, the file-header information may be generated in the ECG- APP during set-up configuration.

When an ECG recording is transferred from the sensor device 1 to the ECG-APP, the actual tags for beat types and arrhythmia may be converted and prepared for transmission from the ECG-APP to the back-end service. The file-header may include a tag MWF_TIM (85h), which defines the correct time of the starting sample within the recordings. This may have as consequence that the pointer will have an offset=0 and are not included in the tags.

In Table 3 an example of the content of appropriate tags is described as may be used in an ECG file of the present invention, wherein all yellow rows contain static information for present invention coded MFER headers. The red colored rows define actual Patient-ID and Investigation-ID and will be static info for an ongoing investigation.

The blue colored rows contain the timestamp of the actual ECG-data, and also defines how many ECG samples are transferred and this needs to be calculated for each file to be transferred based on how many samples are transferred from the Sensor.

The orange colored rows contain detected events, which may be multiples in each file containing both beat-events and rhythm-events, and the INF-tag defining the Rhythm Severity Index which will be set to the highest level detected in the recording period.

The green colored row contains the actual ECG data samples. Each MFER-file may have an END-tag.

The actual severity index tag for that file is included in the orange coded events, and will also be used in the file-name in order to easy arrange the detected arrhythmia episodes.

A use case is exemplified in figure 8A and 8B, wherein the user in the APP GUI will be notified of any arrhythmias detected. If a typical Green arrhythmia event is detected, this will normally not require any medical interventions and an automated report may be provided for the user for download. If a typical Red arrhythmia event is detected, it is recommended to the user to have the findings evaluated by a medical doctor. The user may be given the option of giving the Family doctor access for logging in to a web-service to get an overview of the actual investigation and the arrhythmia findings. Another option provided to the user may be to choose to ask for a web-cardiologist to make the clinical evaluation as a payed service.

Further analysis that may be provided by the sensor device and analysis include for example: respiration and blood pressure. Using machine learning and Al features of a back office toolset may further increase the usability and ability to detect and define diagnosis of irregularities of body functions.

The ECG-APP may be able to download a predefined FHIR Questionnaire from the back-end service for the user to fill in the actual status and clinical condition, and upload the questionnaire response with calculations of the user's medical risk profile to be used by clinicians when evaluating the detected arrhythmia situations.

In another scenario, the questionnaire may be based on an early warning score, where the ECG-APP in an automatic or semi-automatic function based on the actual measured parameters from the sensor, can give a warning in sudden changes in the user's medical situation, where the response can trigger a push notification to personnel at a remote monitoring health service Response Centre.

Normally, the user will need to be within the Bluetooth communication range between the sensor and the portable communication unit 41, such as a smart phone. By measuring the actual signal level, the portable communication unit may give a warning sound and user notification in situations with unsatisfactory signal level.

Such sound alarm may alternatively be given from the sensor by measuring the Bluetooth communication signal level.

The portable communication unit may in the present invention be set up to comprise some or all of the feature discussed above related to the back-end services.

A typical use-case is arrhythmia diagnostics for a user who has placed the sensor/sensor-patch on the chest, and started the actual ECG-APP communicating with the sensor and the back-end services on the back end server.

At start-up, the sensor will set the real-time clock according to the clock in the connected mobile phone running the ECG-APP, and the sensor will start analysing sensor inputs, detecting ECG signals during a warm-up period of 30 seconds, where the ECG-signal quality level is calculated. If the signal quality is acceptable, then the adaptive ECG parameters are calculated and used by the sensor algorithm for hart-beat and rhythm detection. If the signal quality is un-acceptable, the user is instructed to make sure the sensor/senor-patch is correctly placed on the chest, and the warm-up procedure is restarted.

After successful warm-up, a 1-minute reference ECG signal is recorded and transferred to the back-end storage, for example an FHIR storage.

The sensor will regularly (at predefined 4 hour intervals) start 1-minute ECG recording as a control recording.

The sensor arrhythmia detection algorithm will continuously analyse every ECG-signal sample in order to detect a heart-beat, and to analyse for its rhythms. If one of the defined heart-beats Ventricular extrasystole or Supraventricular extrasystole is detected, an event tag is sent to the ECG-APP. If any of the detected arrhythmia events are detected, also such event tags are sent to the ECG-APP.

At the same time, historic ECG samples for 30 seconds are streamed from the sensor to the ECG-APP and this stream continues until 30 seconds after termination of the arrhythmia condition defined when a new normal heart rhythm is detected, wherein the Severity index is sent at the end of the ECG-signal stream. If during this period, a more severe arrhythmia event is detected, the Severity index sent at the end of the ECG-signal stream is updated to the most severe level detected.

All data sent from the sensor to the ECG-APP is automatically forwarded to the back-end service, for example an FHIR service, without any user notification as a Medical Waveform Format file. Thus, ECG recordings are only forwarded upon detected arrhythmias, or at the periodic recording interval, and the stored ECG-data represents discontinuous recordings; however, the ECG recordings comprise a time-stamp defined by the sensor.

In addition, the sensor may calculate RR-intervals continuously and for example every 30. minutes transfer those values to the back-end storage, for example an FHIR storage. Those recordings may be used to visualize a continuous graph of the RR-intervals or Heart-rate, in order to display sudden changes due to arrhythmia events .

As the arrhythmia detection algorithm in the sensor is based on real-time calculations from sample to sample and with limited storage capacity, the arrhythmia analysing time-window is limited to some msec. This can cause detections of possible arrhythmias due to noise disturbances or other artefact situations, False Positive detections. As it is important to avoid False Negative situations where the arrhythmia goes undetected, the acceptable average number of False Positive detections may be relatively high (in the region of 10-20%). Preliminary test results gives approximately 20% False Positive detections of Atrial Fibrillation.

In order to reduce the occurrence of False Positive detections, a post-processing algorithm is implemented, for example in the back-end server.

Arrhythmia event detections sent from the sensor and the ECG-APP to the back-end services will in an **FHIR** storage be coded as Observations with Interpretations as "Preliminary".

On the back-end server, the post-processing algorithm implemented will make use of AI-methods in detection of beats and rhythms. Adaptive parameters are calculated from the first reference ECG-signal recorded, as well as from the periodic ECG-recordings. The algorithm may comprise the following steps:
- Detect too noisy segments not being analysed,
- Detect actual heart-beats.
- From each heart-beat detected, the feature parameters like R-S amplitude, Q-R amplitude, preceding R-R interval, consecutive R-R interval, P-R time, Rhythm, QRS width and Deflection are calculated.
- Use a trained Al-neural network to classify the actual beat as either Normal, Ventricular, Supraventricular or Unclassifiable.

Present invention may also utilize Matched filtering in the meaning of a process for detecting a known piece of signal that is embedded in noise. The filter maximizes the signal to noise ratio of the signal being detected with respect to the noise, and this property makes it suitable for signal recognition. The filtering is accomplished by convolution the input signal with the time-inverted reference template, which represents the shape of the signal the filter is looking for (mathematically the process is an equivalent of a cross-correlation of the input signal with the reference template). Output of the filter shows a peak each time the shape of the input signal looks close to the shape of the reference, and the peak amplitude can be used as a quantitative indication of the similarity.

The optimal shape of the template is the shape of the signal the filter is looking for, so for the purposes of ECG analysts it would be a shape of an ideal QRS complex. The only problem is that the QRS complexes are highly variative; not just from one person to another, but also for the same person during the time. It is practically impossible to choose a single shape that fits all the possible variations.

The adaptive matched filter, implemented in the processing algorithm, exploits the fact that QRS complexes are repetitive and do not change shape too fast. The filter starts a search using a simple artificial QRS-like shape as a template. Each time it detects a peak on the output signal (meaning the input signal has similarity to the template), it adjusts the template using the input signal from that point in time. The majority of the peaks comes from QRS complexes, so each time a QRS complex is detected, the template transforms more and more to the shape of an averaged QRS complex, the complex that is specific for the particular person at the particular time and condition.

Additional constraints and rules are added to the algorithm to minimize effect of template updates caused by definitive noise and imperfections. In general the rules quantitatively determine on per-QRS basis how much the detected QRS is allowed to influence the shape of the template. The rules use our knowledge about the ECG signal in terms of timing, regularity and repeatability. They help to achieve a robust lock on the QRS complexes and disregard the artifacts.

Long time-series of R-R-interval variations can be calculated based on the continuous recording and storage of R-R-intervals separated from the 1-minute ECG-recordings as an anomaly detector. Based on the variance in the RR-interval, the algorithm will analyse the data looking for possible arrhythmia events.

If the analysis detect a corresponding arrhythmia event result compared to the "Preliminary" result obtained by the sensor, the Interpretation of the arrhythmia event is changed to "Final".

But, if the analysis detect a different arrhythmia event result compared to the "Preliminary" result obtained by the sensor, the arrhythmia event result is changed in the FHIR server and the Interpretation is changed to "Final", but also with an additional "Corrected" remark linked to the Interpretation.

When the ECG-APP is searching in the back-end storage, for example the FHIR storage, looking for results to be displayed to the user, only "Final" results are displayed. The post-processing algorithm starts immediately after a "Preliminary" detected arrhythmia event is transmitted to the back-end storage, for example the FHIR storage, and takes only a few msec, normally < 500ms. With normal processing time, the user will experience little or no delay, and the "Final" result is available and presented in what is experienced by the user as in real-time.

Preliminary test results show 20% False Positive AF detections from the sensor arrhythmia detection algorithm, and this can probably be reduced to less than 10% by use of the post-processing arrhythmia detection algorithm.

Al methods can be based on use of Neural Networks, which has to be trained by the use of standard ECG databases like the MIT databases from Physionet.

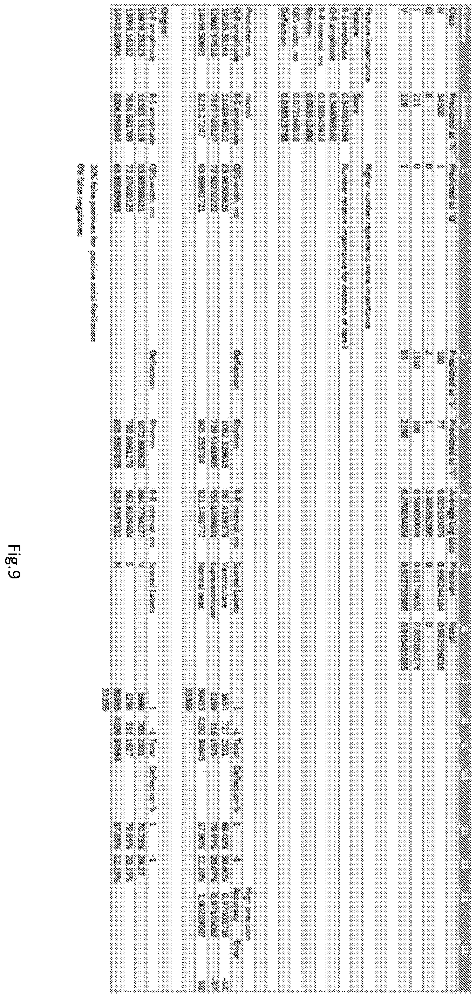

Preliminary results, illustrated by the data log in figure 9, based on the databases MIT-BIH Supraventricular Database (32 records) and MIT-BIH Arrhythmia Database (32 records) where 70% of the data are used for training and 30% as test data shows that a fully connected Neural Network consisting of 300 hidden nodes, a learning rate of 0.02 and with 500 iterations, can use extracted parameters like Q-R amplitude, R-S amplitude, QRS width, Deflection, Rhythm, R-R interval, and gives a predictive average accuracy for N, S, V and Q types of beat at a level of 0.99, with a predicted accuracy for N beats of 99,1%, V beats of 92.1% and S beats of 80,5%.

Data extracted from the sensor, the ECG-APP, and the back-end storage, for example an FHIR storage, may be used for further training of the Neural Networks, an thus be used to provide improved device and services features related to the Al modules.

The present invention may use Al and ML methods in the arrhythmia detection software/firmware/hardware..

The ECG analysing program will detect actual heartbeats, and calculating parameters identifying a heart-beat. Those parameters are analysed by the implemented ML-algorithm in order to determine which type of hart-beat that correspond to the parameters as discussed below. The beat-type and corresponding beat parameters are stored in an Annotation file, to be used for the Rhythm detection part of the analysing program.

The training process for the ML-program is a continuous process used both for the individual user/patient, but will also be used across all patients based on the pool of collected ECG data from all users.

The ML algorithm is designed to predict abnormal beats with high accuracy, using a two-step process consisting of both un-supervised learning and supervised learning procedures. The un-supervised part of the algorithm will be able of distinguishing between Normal beats and Abnormal beats. If an Abnormal beat is detected, there will be a parameter extraction as described for the adaptive process, and a supervised learning algorithm will be used to distinguish which type of abnormal beats are detected. This can for instance be a VES or SVES type of beat, an artefact beat or any other Irregular beats.

A detected Irregular type of beat will be flagged and should be manually verified and corrected if necessary to be correctly stored in the actual Annotation file. All manually corrected beats are used in a feedback-loop to the supervised learning procedure in order to be a continuous learning process.

The reason for implementing such AI and ML methods is to obtain a reasonable low level of False Positive (FP) arrhythmia events, and at the same time guaranties for a low level of False Negative (FN) detections. As those goals are in statistical conflict, there will always be a balance between FN and FP and it may be difficult to obtain an automated arrhythmia detection software with sufficiently low levels for both parameters.

In some embodiments of present invention it is defined an acceptable level of FN of 0,5% with a goal of obtaining FP < 5%. By implementing ML methods, it might be possible to achieve such goals, and this description of present invention describes elements and features comprised in new ML algorithms to be implemented into the embodiments of present invention.

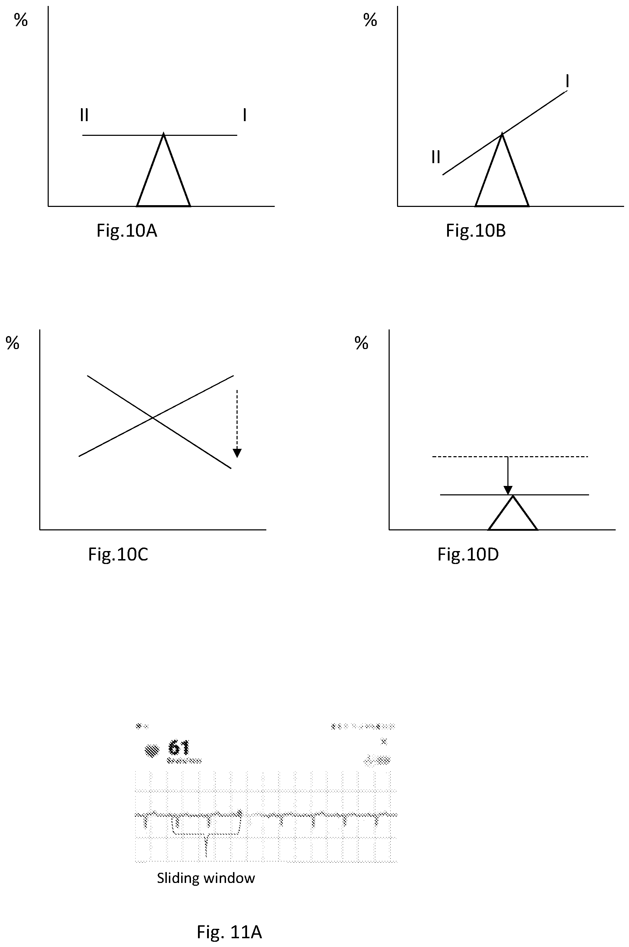

Implementation elements of Machine Learning, ML, and other aspects related to figures 10-14 will now be discussed. The various aspects of the data processing may be performed in a back office service provided in a cloud based network setting, but could also be implemented in an ECG-APP, or in the device itself. Thus, the discussed features related to data analysis should not be limited to the example environment discussed, but understood as could be equally implemented on the various modules of the present invention, either wholly or in part. Such that one can also expect the various processing part s of a complex system to share processing resources to accomplish best possible result, in real time as in post processing and giving feedback to any of patient and authorized 3.rd person/system.

In figure 10A to 10E the balance between False Negative, FN, and False Positive, FP, is discussed.

When developing algorithms for arrhythmia detection, there will statistically be a balance between False Positive (FP) I and False Negative (FN) II. This is called Type I and Type II errors, see figure 10A.

In the sensor Firmware, it is of outmost importance to avoid FN arrhythmia events; thus, the number of FP will increase, as seen in figure 10B. This will normally not be of any problem, there will be several in-correct arrhythmia events detected and transferred to the back-end service but at the end-user is not notified this is acceptable.

As the post-processing algorithm will analyse detected events to determine if this has been a real arrhythmia event or caused by artefacts, the post-processing algorithm is designed to decrease the FP detected events as illustrated in the figure 10C. This 2-step principle can be expected to give as result, a lowered level of errors both of Type I and Type II compared to a single algorithm, as illustrated in the figure.

If the 2 algorithms are designed on the same principles for detecting heartbeats and arrhythmias, the achieved benefits will probably be marginal.

However, of the post-processing algorithm can use a more through analyse of both beat detections, beat classifications and analyses for arrhythmias over a longer time-window than what is possible in the sensor, the results can probably lower the ration of FP with a minimum of increased number of FN, see figure 10D.

So, the focus in the post-processing algorithm is to filter out detected arrhythmia episodes that are not real arrhythmias which can be caused by artefact detections or disturbances due to physical activities. What will be of importance for quality control is to evaluate arrhythmia episodes filtered out by the post-processing algorithms in order to verify if any real arrhythmia episodes are removed because this will be a FN situation.

The challenge for the post-processing algorithm will thus be to filter out as many as possible of the artefact detections without introducing FN situations.

In present invention, the algorithm implemented in the electrical device 3 is based on teh use of discrete mathematics and real-time analysing both for beat detection and for arrhythmia detection within a narrow timeframe. The algorithm may be implemented in Firmware, Hardware or other. It is expected that over time more dedicated chipsets will be provided and then can be implemented to optimize cost aspects, implementation aspects, and production aspects.

As the electrical device 3 and sensor disposable patch 2 samples ECG-data at for example 256 Hz, which gives about 3,9 msec between each sample, all calculations should preferably be executed during this time. At the same time, it may be limited space for storage of historic data, and for comparing signals in the beat detection and the arrhythmia detection part of the algorithm. A narrow "sliding-window" principle may be used for the detections, as illustrated in the figure 11A.

On the other hand, the post-processing algorithm may be provided with a file comprising for example 1-4 minutes of ECG recordings. Post-processing services may then use repeated times analyses of this file both for reliably detection of beats and analysing for arrhythmias. Thus, there may be deployed different algorithm methods and this combination gives possibilities for lowering the number of FP without increasing the number of FN detections.

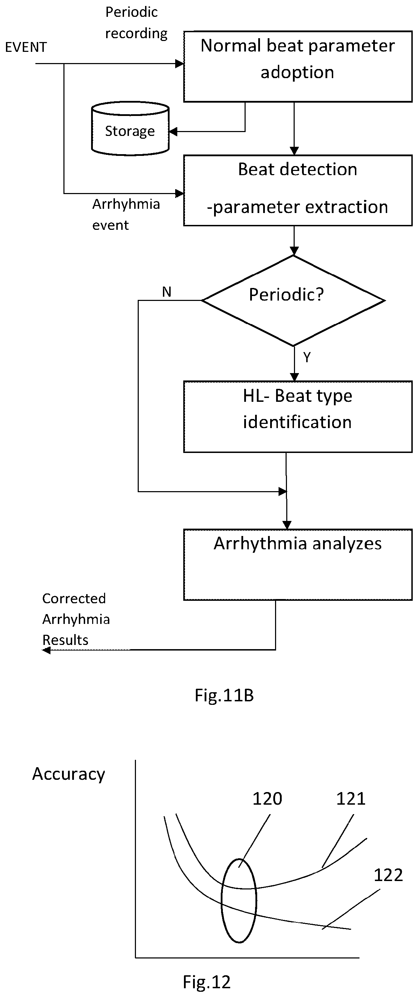

Now considering the post-processing algorithm as exemplified in figure 11B, and figure 5, several different steps are taken. First the actual file, comprising the event, is analysed in a beat detection and adoption process. Here it is provided the task of adopting the algorithm to the specific ECG signal from the person, and to reliably detect Normal beats. It is assumed that the first Periodic recording will be a relatively noise-free recording as the patient will be seated, and still, during the start-up of a new investigation. The adaptive parameters may be stored in SMILE, or equivalent, and used in every instance of the Beat detection module. For the consecutive periodic recordings, the actual parameters are updated.

The first step in analysing an arrhythmia event is here the Beat detection, where each beat is identified at the R-peak. From the sampled ECG-curve values, typical beat parameters are calculated as characteristic parameters for the beats, such as, but not limited to:
- QRS amplitude
- V amplitude
- R-R interval
- Rate
- QRS likehood
- V width
- V deflection

In principle, those extracted parameters may be used for precise identification of the actual type of beat.

It is an aim of the ML-method used in present invention to provide identification of beat types. Normal beats are the first type to be detected, and may be used for detection of normal beats for the Periodic recordings. The output of the ML-method may comprise identification of each beat with correct type including artefact beats coded as "|". The output of beat types may be used as input to the Arrhythmia analyser. Several sophisticated analyses are repeated in order to reliably detect the actual arrhythmia type.

The output from the Arrhythmia analyser will make necessary corrections in the stored parameters in SMILE CDR or similar data repository. In principal, the actual event is either confirmed or rejected. A rejected arrhythmia event may be classified as "Low Signal Quality" and not shown to the end-users. However, the Cardiologist may access these in order to evaluate those files to analyse for possible FN situations.

Present invention was tested in an early phase beta-test, and achieved the model fit with performance shown in the table 3 below. As can be seen, Normal beats were reliably detected (99,0%) whereas other beat types was less reliably detected. More training data from a plurality of users and different rhythm conditions will provide necessary improvements in the performance.

Notation in figure: normal beat (N), ventricular ectopic beat (V), supraventricular ectopic beat (S), fusion beat (F), and unknown beat (|).

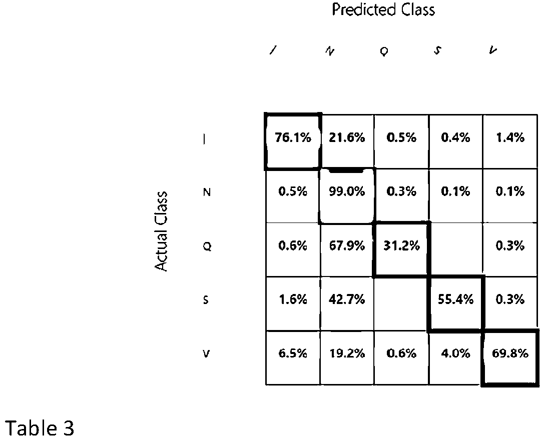

An ML-model will normally be developed as a trained model, where manually annotated training data is used for the repeated iterations in the algorithm training procedures. By fine-tuning through several iterations, the model can be trimmed to give good match to the training dataset.

However, as seen in figure 12, when the same model is used for analyzing real-world data 121, a well-known optimal training fit 120 can be achieved, whereas more training of the model will give a distinct decrease in performance 122. It is therefore necessary with repeated training and testing to achieve an optimal configuration of the ML-model.

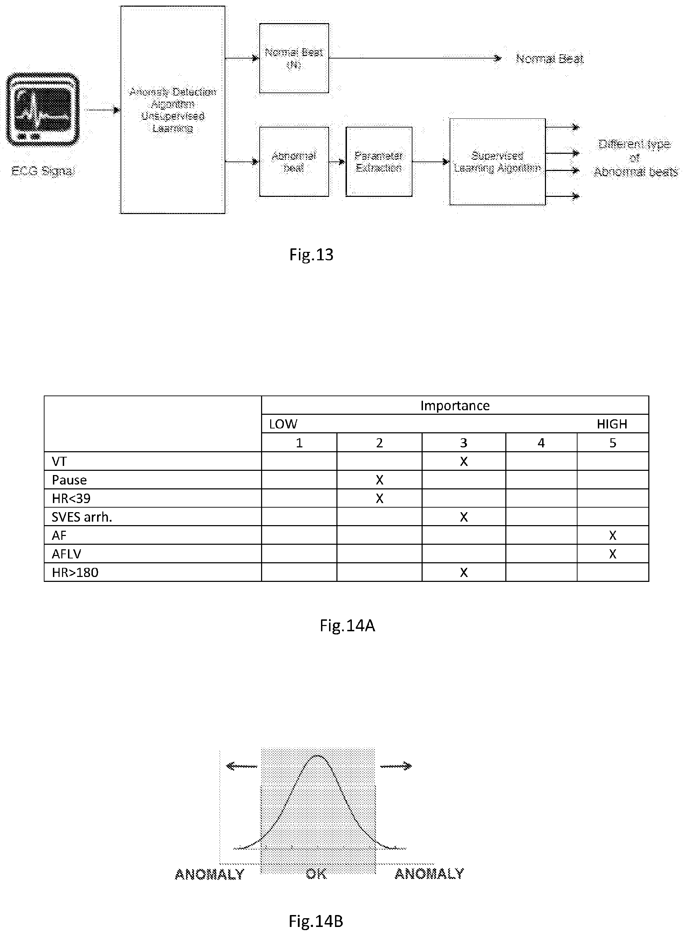

A new ML algorithm is provided to predict abnormal beats with high accuracy, as outlined in figure 13. To achieve this, a two-step machine learning algorithm is built, which will use both unsupervised and supervised ML algorithms. The building process comprise:
- Step 1: detecting the abnormal beats using the anomaly detection algorithm such as auto-encoders
- Step 2: extracting the parameters of abnormal beats and label them manually
- Step 3: using a supervised learning algorithm to classify different abnormal beats.

For manual labelling, the task may be minimized as it is not required to look into the whole ECG signal to label the beats. Only beats detected as abnormal will be flagged in the ECG signal, which in most cases compromises a maximum of 10 % of beats, patient dependent.

The supervised learning algorithm will be able of detecting S, V and Q beats as well as identifying most of the artefact beats "|". If a beat does not fit into any of the defined beat types, this beat will be flagged as anomaly, but most likely this is an artefact disturbance.

In this model, it may be provided possibilities for storing patient-specific parameters identifying a normal beat similar to existing solution, in order to re-use the actual parameters when analyzing the next detected arrhythmia event. This implies a gradually fine-tuning of the unsupervised learning model for better performance during a test period.

In deployed discrete algorithms all parameters for arrhythmia detections are hard-coded, with no possibilities for manually corrected cut-off values. It is however outlined a new regime for arrhythmia detection situation, in which it may be a prerequisite to make manually adjustments, for example wherein some parameters are changed at start-up of an investigation.

In order for a doctor/user to individually adjust parameters for desired types of arrhythmias, this can be achieved by implementing a parameter defining the Importance of the actual type of arrhythmia. This can for instance be within defined steps between 1-5.

As the ML-algorithms will be able of detecting arrhythmias and distinguish a real arrhythmia from anomaly situations, usually defined as a Normal distribution of parameters/likelihood. If for instance if the actual patient is not suspicious to have VES types of beats, it may be of no clinical importance to detect single VES-types of beats. It can be tolerable to accept a high number of FN for the VES-types and the Importance parameter can be defined to a low level of 1. If it is of high importance to detect AF-episodes, it can be tolerable to accept a higher number of FP episodes and the Importance parameter can be set to a high level of 5. The Importance parameter is used for the cut-off values in the ML-algorithm for anomaly detection. This method is based of statistically likelihood and will manipulate the area of acceptable limits for a normal distribution of parameters. It is supposed to be an efficient way for doctors to fine-tune the characteristic of the arrhythmia detection software.

Fine-tuning an individual set-may comprise making configurations of the importance in the actual arrhythmias for the actual patient. All fine-tuning of the arrhythmia detection parameters will have consequences for the FN and FP detection rate. This is exemplified in figure 14A wherein the actual focus is to detect (atrial fibrillation) AF situations while it is less likely that a (supraventricular extrasystoles) SVES arrhythmia episode shall occur, and further, it can be tolerable to accept a higher FN for the SVES arrhythmias while it can be necessary to accept a higher FP rate for AF detections.

In this example, Low importance will have as consequence that FN situations can be increased because this is not so important for that patient, while High importance will give increased number of FP but will increase the chance of detecting even shorter episodes of AF.

In the ML-model the implications will be to change the cut-off values for anomaly detections. This can be at first an experimental study where the parameter in the unsupervised learning algorithm is adjusted depending on patients. For critical patients, it may be advantageous to decrease the area illustrated by the grayed out region of the beat in figure 14B. This means it may be possible to capture even slight deflection in the ECG.

Examples of actual use-cases embodiments and technology implementations are briefly discussed below, and should be understood as non-limiting examples:

### Personal arrhythmia monitoring

The technology may be used based on a private initiative, where a user can buy the technology (device/app) from a pharmacy or web-shop and start up an arrhythmia investigation. In cases where arrhythmias are detected, the user can choose to give data access to a clinician by for example providing and giving a secret code generated in the user's APP. Upon the clinicians request for access in the WEB-service, a push notification may be forwarded to the user telling that a named clinician request access to the users data. The user may then reject or accept this request, and if accepted this will be a digital consent of access whereas the clinician is given permission for accessing the stored ECG data. The user may in the APP interface have a list of all persons having been granted access, and may thereafter at any time withdraw this access causing the permissions for that person to be terminated.

### Holter-investigation at public and private healthcare services

The controlling software may be optimized for use by a public or private healthcare clinic, where it is possible to integrate the back-end services into the Electronic Healthcare Record systems. Upon a doctors decision, an device according to present invention can be associated with a defined patient identified by his/her social insurance number. A push message to his/her smartphone may be used for electronic consent to shared access to recorded data.

### Cardiac Telemetry Monitoring

In the description, it is detailed how it is possible to have a real-time visual display of recorded ECG-data directly in the Smartphone display. It is also possible to forward this information to a dedicated streaming server software in order to forward this information in near-real-time directly to a display station in a cardiac monitoring service. Such functions can be similar to existing radio-transmission based cardiac telemetry services used in hospitals. The actual data transfer may be based on public mobile data transfer or hospital-internal wireless communication system. As the transmission from the patient's Smartphone by default is based on public mobile data transfer, such monitoring services may be used independent on the patient's location and can thus be used for remote home monitoring purposes. By implementing the GPS positions detected in the Smartphone, the clinicians can also monitor the patient's location in cases of emergencies.

### Local data storage in the sensor and/or Smartphone

By default, the device of present invention will transfer to a Smartphone detected arrhythmia episodes, and the data will automatically be forwarded to the back-end data storage. In cases where the Bluetooth communication between the ECG sensor and the Smartphone is interrupted, the ECG sensor can have implemented Flash memory in order to temporary storage of several hours of recorded ECG data. When the Bluetooth communication is re-established, the actual temporary stored ECG data can automatically be forwarded to the Smartphone.

Such functionality can give possibilities of arrhythmia detection functions independent of a Smartphone when an investigation have been started, and the user may at any time connect to a Smartphone or similar device for uploading to the back-end services all recorded data and detected ECG arrhythmia episodes. The Smartphone can temporary store recorded ECG data in cases of disruption in the mobile data transfer, and with automatically forwarding of data when the communication is reconnected. This will allow for arrhythmia detection services also in areas with no mobile phone coverage.

### Emergency care services

The Smartphone APP software may be implemented directly in a vital signs monitoring device with implemented Bluetooth communication. This will allow for real-time display of recorded ECG graph and with arrhythmia detected warning signals, to be used in emergency care services in ambulances or rescue operation services in helicopters and airplanes.

### Sensor with smartphone functionality implemented

It is further possible to implement mobile data communication functionalities directly into the device of present invention in a similar way as smart-watches using e-SIM identification or similar solutions. This will allow for implementing the described APP functionalities directly in the device, thus giving possibilities of ECG monitoring and arrhythmia detection services independent of a Smartphone. Actual mobile data communications can be 4G, 5G, WiFi or similar services.

## Claims

1. An electrocardiogram, ECG, sensor device (1) for wireless biopotential measurement on a person or object skin/surface, comprising:
a disposable patch (2) comprising a base portion of a connector (12),
a battery (13) arranged in the base portion of the connector (12), and
a reusable electronic device (3) connectable to the base portion of the connector (12),
a cover (14), for protecting the internal components of the electronic device,
wherein the disposable patch (2) comprises a multilayered flexible assembly having a lower portion (32) and an upper portion (31), the lower portion (32) comprising at least:
a first conductive layer (26) comprising pick-up electrodes (21, 22) for measuring biopotential level at the person or object skin/surface, and a shielding electrode (23),
wiring (17, 18, 19) connecting the pick-up electrodes and the shielding electrode (23) to a connector (16),
the electrocardiogram sensor device further comprising: shielding layers (25, 27, 28) arranged above and under the wiring layer,
a dielectric shielding layer (25) arranged under the wiring layer, two dielectric shielding layers arranged above the wiring layer and covering the whole area of the disposable patch (2) with the exception of the area of the underlying shielding electrode (23),
a second layer of conductive material (29) arranged above the dielectric layers (27, 28) covering at least the area above the underlying layers comprising the pick-up electrodes (21, 22) and the shielding electrode(s) (23),
wherein the shielding electrode (23) is in contact with the person or object skin/surface and is electrically connected to the second layer of conductive material (29) via apertures in the dielectric layers (27, 28),
the reusable electronic device (3) comprising an electronic print-card (35) connected to the shielding electrode (23) via a connector, thereby providing electrostatic shielding (45), and
the upper portion (31) of the disposable patch (2) being foldable around the battery (13) such that the connector (16) faces upward for connection to a second connector (34) of the reusable electrical device (3).

2. Sensor device according to claim 1, wherein the pick-up electrodes (21, 22) are wireless sensors comprising a circuit which is floating with respect to a ground potential.

3. Sensor device according to claim 1 or 2, wherein the distance between the pickup electrodes (21, 22) is less than 10 cm, or less than 8, or less than 6 cm.

4. Sensor device according to any of the previous claims, wherein
the electronic device (3) and the pick-up electrodes (21, 22) are arranged longitudinally along the central line (*cl*), and wherein the electronic device (3) is attached to the patch (2) in a first end, and the pick-up electrodes (21, 22) at different positions along a center line (*cl*) towards the other end of the patch (2), and
the wiring (18, 19) connecting each of the pickup electrodes (21, 22) to the electronic device connector (16) are kept substantially equal in length and in close distance to each other in a substantially symmetrical design, thereby reducing footprint area and vulnerability to pickup of signal noise.

5. Sensor device according to any of the previous claims, wherein the electronic device comprise:
- a signal amplification module connected to the pick-up electrodes (21, 22) on its input side,
- an A/D digitalization module connected to the signal amplification module on its input side,
- a storage module storage module for storing sampled data being connected to the A/D digitalization module on its input side,
- a wireless communication module for communicating data to and from a remote computer resource, and
- a signal analyzer for analyzing the sampled data being connected to the A/D digitalization module and or the storage module on its input side, and
- a feedback gain adjustment signal being output from storage module or signal analyzer and fed into the A/D digitalization module.

6. Sensor device according to any of the previous claims, further comprising:
a noise-pick-up electrode (103) located between the shielding layers (27, 28) above the wiring layer, and positioned longitudinally between the pick-up electrodes (21, 22),
the noise-pick-up electrode (103) is connected by wiring to A/D digitalization module in anti-phase to the measured ECG-signal in order to provide a noise cancelling mechanism.

7. Sensor device according to any one of the previous claims, wherein the cover (14) having a semi-conducting material (45) sprayed on the inside.

8. Sensor device according to any one of previous claims, wherein shielding electrode (23) is arranged on the patch (2) to be arranged under the position of the electronic device (3).

9. Sensor device according to any one of previous claims, wherein the electronic device (3) further comprise:
an arrhythmia analyzer detector module for arrhythmia analyzes, and a severity index estimator module, wherein the output from arrhythmia analyzer detector is used for severity estimation according to a predefined severity index table.

10. Sensor device according to any one of previous claims, wherein the electronic device (3) further comprise:
an impedance imbalance detector providing a variable impedance for attenuating the impedance imbalance on the signal amplifier input, wherein variable resistors 101, 102 is provided between the pick-up electrodes and the signal amplifier, and wherein the variable impedance is provided by the variable resistors 101, 102 being software controlled.

11. Sensor device according to claim 10, further comprising one or more noise-pick-up electrodes (103) arranged between the layers of dielectric ink (27, 28) for providing input signal for noise cancelling modules, and wiring connecting the noise-pick-up electrodes (103) to the connector (16).

12. Sensor device according to any one of previous claims, wherein the electronic device (3) further comprise an Al module trained and configured to:
- detect arrhythmia episodes in the detected ECG signal, and/or
- detect and differentiate between arrhythmia and signal noise caused by external influences or natural variances of hart activities related to physical or emotional variances, and
- the Al module is further configured to provide input to a noise attenuating module providing a noise cancelling signal on the signal amplifier input.

13. Method for wireless biopotential measurement using an electrocardiogram, ECG, sensor device (1) according to any claims 1-12, comprising:
- arranging and fastening the plaster patch on the breast bone with the longitudinal central line aligned with the breast bone, and with the electronic device (3) in the uppermost position,
- attaching the electronic device (3),
- activating a measurement routine,
- the electronic device (3) receiving biopotential readings from the pick-up electrodes (21, 22), and transmitting the sensor data wirelessly to a computer device (41) and optionally to a back-end server (42).

14. Method for wireless biopotential measurement according to claim 13, wherein the analysis of sensor device data using a trained Al-system based on neural network models, and
wherein the trained Al-system based on neural network models resides in any of the electronic device (3), the computer device (41), or the back-end server (42).

15. System for wireless biopotential measurement, the system comprising:
- an electrocardiogram, ECG, sensor device (1) according to any one of claim 1 to 12,
- a computer device (41) being in communication with the sensor device (1) over a wireless communication line, and
- a computer implemented analyzing program, and
the system further comprising a back-end server (42) being in communication with the computer device (41) over a wireless communication line, wherein the back-end server provides one or more of the following services for the computer device (41) and the sensor device (1):
- uploading sensor device data,
- analyzing sensor device data,
- analyzing sensor device data using a trained Al-system based on neural network models,
- providing result for display on computer device (41),
- communicating results to computer device (41), and
- communicating results to one of user's WEB or Doctor's WEB.

## Patentansprüche

1. Elektrokardiogramm-Sensorvorrichtung, EKG-Sensorvorrichtung, (1) zur drahtlosen Biopotentialmessung auf der Haut/Oberfläche einer Person bzw. eines Objekts, umfassend:
ein Einwegpflaster (2), umfassend einen Basisabschnitt eines Verbinders (12),
eine Batterie (13), die in dem Basisabschnitt des Verbinders (12) angeordnet ist, und
eine wiederverwendbare elektronische Vorrichtung (3), die mit dem Basisabschnitt des Verbinders (12) verbindbar ist,
eine Abdeckung (14) zum Schützen der inneren Komponenten der elektronischen Vorrichtung,
wobei das Einwegpflaster (2) eine mehrschichtige flexible Baugruppe umfasst, die einen unteren Abschnitt (32) und einen oberen Abschnitt (31) aufweist, wobei der untere Abschnitt (32) mindestens Folgendes umfasst:
eine erste leitfähige Schicht (26), umfassend Abgriffselektroden (21, 22) zum Messen des Biopotentialniveaus auf der Haut/Oberfläche der Person bzw. des Objekts und eine Abschirmelektrode (23),
Verdrahtung (17, 18, 19), welche die Abgriffselektroden und die Abschirmelektrode (23) mit einem Verbinder (16) verbindet,
wobei die Elektrokardiogramm-Sensorvorrichtung ferner Folgendes umfasst: Abschirmschichten (25, 27, 28), die über und unter der Verdrahtungsschicht angeordnet sind,
eine dielektrische Abschirmschicht (25), die unter der Verdrahtungsschicht angeordnet ist,
zwei dielektrische Abschirmschichten, die über der Verdrahtungsschicht angeordnet sind und den gesamten Bereich des Einwegpflasters (2) unter der Ausnahme des Bereichs der darunterliegenden Abschirmelektrode (23) abdecken,
eine zweite Schicht leitungsfähigen Materials (29), das über den dielektrischen Schichten (27, 28) angeordnet ist, welche mindestens den Bereich über den darunterliegenden Schichten, umfassend die Abgriffselektroden (21, 22) und die Abschirmelektrode(n) (23), abdecken,
wobei die Abschirmelektrode (23) in Kontakt mit der Haut/Oberfläche der Person bzw. des Objekts steht und über Öffnungen in den dielektrischen Schichten (27, 28) elektrisch mit der zweiten Schicht leitungsfähigen Materials (29) verbunden ist,
wobei die wiederverwendbare elektronische Vorrichtung (3) eine elektronische Druckkarte (35) umfasst, die über einen Verbinder mit der Abschirmelektrode (23) verbunden ist, wodurch eine elektrostatische Abschirmung (45) bereitgestellt wird, und
wobei der obere Abschnitt (31) des Einwegpflasters (2) derart um die Batterie (13) klappbar ist, dass der Verbinder (16) nach oben zur Verbindung mit einem zweiten Verbinder (34) der wiederverwendbaren elektrischen Vorrichtung (3) gerichtet ist.

2. Sensorvorrichtung nach Anspruch 1, wobei die Abgriffselektroden (21, 22) drahtlose Sensoren sind, umfassend einen Schaltkreis, welcher in Bezug auf ein Erdungspotential schwebt.

3. Sensorvorrichtung nach Anspruch 1 oder 2, wobei der Abstand zwischen den Abgriffselektroden (21, 22) kleiner als 10 cm, oder kleiner als 8 oder kleiner als 6 cm, ist.

4. Sensorvorrichtung nach einem der vorhergehenden Ansprüche, wobei
die elektronische Vorrichtung (3) und die Abgriffselektroden (21, 22) in Längsrichtung entlang der Mittellinie (*cl*) angeordnet sind, und wobei die elektronische Vorrichtung (3) in einem ersten Ende an dem Pflaster (2) befestigt ist, und die Abgriffselektroden (21, 22) an unterschiedlichen Positionen entlang einer Mittellinie (*cl*) in Richtung des anderen Endes des Pflasters (2) angeordnet sind, und
die Verdrahtung (18, 19), die jede der Abgriffselektroden (21, 22) mit dem elektronischen Vorrichtungsverbinder (16) verbindet, im Wesentlichen gleich lang und im engen Abstand zueinander in einer im Wesentlichen symmetrischen Ausgestaltung gehalten werden, wodurch der Fußabdruckbereich und die Anfälligkeit zur Aufnahme von Signa-Geräuschen reduziert wird.

5. Sensorvorrichtung nach einem der vorhergehenden Ansprüche, wobei die elektronische Vorrichtung Folgendes umfasst:
- ein Signalverstärkungsmodul, das mit den Abgriffselektroden (21, 22) auf seiner Eingangsseite verbunden ist,
- ein A/D-Digitalisierungsmodul, das mit dem Signalverstärkungsmodul auf seiner Eingangsseite verbunden ist,
- ein Speichermodul Speichermodul zum Speichern erfasster Daten, das mit dem A/D-Digitalisierungsmodul auf seiner Eingangsseite verbunden ist,
- ein drahtloses Kommunikationsmodul zum Kommunizieren von Daten an eine und aus einer Remote-Computerressource, und
- einen Signalanalysator zum Analysieren der erfassten Daten, der mit dem A/D-Digitalisierungsmodul und oder dem Speichermodul auf seiner Eingangsseite verbunden ist, und
- ein Rückkopplungsverstärkungs-Anpassungssignal, das aus einem Speichermodul oder Signalanalysator ausgegeben und in das A/D-Digitalisierungsmodul eingespeist wird.

6. Sensorvorrichtung nach einem der vorhergehenden Ansprüche, ferner umfassend:
eine Geräusch-Abgriffselektrode (103), die sich zwischen den Abschirmschichten (27, 28) über der Verdrahtungsschicht befindet und in Längsrichtung zwischen den Abgriffselektroden (21, 22) positioniert ist,
wobei die Geräusch-Abgriffselektrode (103) durch Verdrahtung zum A/D-Digitalisierungsmodul in Antiphase mit dem gemessenen EKG-Signal verbunden ist, um einen Geräuschunterdrückungsmechanismus bereitzustellen.

7. Sensorvorrichtung nach einem der vorhergehenden Ansprüche, wobei die Abdeckung (14) ein Halbleitermaterial (45) aufweist, das auf der Innenseite aufgesprüht ist.

8. Sensorvorrichtung nach einem der vorhergehenden Ansprüche, wobei die Abschirmelektrode (23) auf dem Pflaster (2) angeordnet ist, um unter der Position der elektronischen Vorrichtung (3) angeordnet zu sein.

9. Sensorvorrichtung nach einem der vorhergehenden Ansprüche, wobei die elektronische Vorrichtung (3) ferner Folgendes umfasst:
einen Arrhythmie-Analysator-Detektormodul für Arrhythmie-Analysen und ein Modul zur Schätzung des Schweregradindex, wobei die Ausgabe aus dem Arrhythmie-Analysator-Detektor zur Schätzung des Schweregrads gemäß einer vordefinierten Schweregradindextabelle verwendet wird.

10. Sensorvorrichtung nach einem der vorhergehenden Ansprüche, wobei die elektronische Vorrichtung (3) ferner Folgendes umfasst:
einen Impedanzungleichgewichtsdetektor, der eine variable Impedanz zum Dämpfen des Impedanzungleichgewichts an dem Signalverstärkereingang bereitstellt, wobei variable Widerstände 101, 102 zwischen den Abgriffselektroden und dem Signalverstärker bereitgestellt sind, und wobei die variable Impedanz durch die variablen Widerstände 101, 102 bereitgestellt ist, die softwaregesteuert sind.

11. Sensorvorrichtung nach Anspruch 10, ferner umfassend eine oder mehrere Geräusch-Abgriffselektroden (103), die zwischen den Schichten dielektrischer Tinte (27, 28) zum Bereitstellen eines Eingangssignals für Geräuschunterdrückungsmodule angeordnet sind, und eine Verdrahtung, welche die Geräusch-Abgriffselektroden (103) mit dem Verbinder (16) verbindet.

12. Sensorvorrichtung nach einem der vorhergehenden Ansprüche, wobei die elektronische Vorrichtung (3) ferner ein trainiertes KI-Modul umfasst und dazu konfiguriert ist:
- Arrhythmie-Episoden in dem detektierten EKG-Signal zu detektieren, und/oder
- eine Arrhythmie und ein Signalgeräusch, die bzw. das durch äußere Einflüsse verursacht wird, oder natürliche Varianzen von Herzaktivitäten in Bezug auf physische oder emotionale Varianzen zu detektieren oder zwischen diesen zu unterscheiden, und
- das KI-Modul ist ferner dazu konfiguriert, einen Eingang in ein Geräuschdämpfungsmodul bereitzustellen, das ein Geräuschunterdrückungssignal an dem Signalverstärkereingang bereitstellt.

13. Verfahren zur drahtlosen Biopotentialmessung unter Verwendung einer Elektrokardiogramm-Sensorvorrichtung, EKG-Sensorvorrichtung, (1) nach einem der Ansprüche 1-12, umfassend:
- Anordnen und Befestigen des Pflaster-Patches an dem Brustknochen, wobei die längliche Mittellinie mit dem Brustknochen ausgerichtet ist, und mit der elektronischen Vorrichtung (3) in der obersten Position,
- Befestigen der elektronischen Vorrichtung (3),
- Aktivieren einer Messroutine,
- wobei die elektronische Vorrichtung (3) Biopotentiallesungen von den Abgriffselektroden (21, 22) empfängt und Sensordaten drahtlos an eine Computervorrichtung (41) und optional an einen Backend-Server (42) überträgt.

14. Verfahren zur drahtlosen Biopotentialmessung nach Anspruch 13, wobei die Analyse von Sensorvorrichtungsdaten unter Verwendung eines trainierten KI-Systems basierend auf neuronalen Netzwerkmodellen, und
wobei das trainierte KI-System basierend auf neuronalen Netzwerkmodellen in einer beliebigen der elektronischen Vorrichtung (3), der Computervorrichtung (41) oder des Backend-Servers (42) liegt.

15. System zur drahtlosen Biopotentialmessung, wobei das System Folgendes umfasst:
- eine Elektrokardiogramm-Sensorvorrichtung, EKG-Sensorvorrichtung, (1) nach einem der Ansprüche 1 bis 12,
- eine Computervorrichtung (41), die über eine drahtlose Kommunikationsleitung mit der Sensorvorrichtung (1) in Verbindung steht, und
- ein computerimplementiertes Analyseprogramm, und
wobei das System ferner einen Backend-Server (42) umfasst, der über eine drahtlose Kommunikationsleitung mit der Computervorrichtung (41) in Verbindung steht, wobei der Backend-Server einen oder mehrere der folgenden Dienste für die Computervorrichtung (41) und die Sensorvorrichtung (1) bereitstellt:
- Hochladen von Sensorvorrichtungsdaten,
- Analysieren von Sensorvorrichtungsdaten,
- Analysieren von Sensorvorrichtungsdaten unter Verwendung eines trainierten KI-Systems basierend auf neuronalen Netzwerkmodellen,
- Bereitstellen eines Ergebnisses zur Anzeige auf einer Computervorrichtung (41),
- Kommunizieren von Ergebnissen an eine Computervorrichtung (41), und
- Kommunizieren von Ergebnissen an eines vom WEB des Benutzers oder vom WEB des Arztes.

## Revendications

1. Dispositif de capteur d'électrocardiogramme, ECG, (1) pour une mesure de biopotentiel sans fil sur la peau/surface d'une personne ou d'un objet, comprenant :
un patch jetable (2) comprenant une partie de base d'un connecteur (12),
une batterie (13) agencée dans la partie de base du connecteur (12), et
un dispositif électronique réutilisable (3) reliable à la partie de base du connecteur (12),
un couvercle (14), pour protéger les composants internes du dispositif électronique,
dans lequel le patch jetable (2) comprend un ensemble flexible multicouche ayant une partie inférieure (32) et une partie supérieure (31), la partie inférieure (32) comprenant au moins :
une première couche conductrice (26) comprenant des électrodes de détection (21, 22) pour mesurer le niveau de biopotentiel au niveau de la peau/surface d'une personne ou d'un objet, et une électrode de blindage (23),
un câblage (17, 18, 19) reliant les électrodes de détection et l'électrode de blindage (23) à un connecteur (16),
le dispositif de capteur d'électrocardiogramme comprenant en outre : des couches de blindage (25, 27, 28) agencées au-dessus et en dessous de la couche de câblage,
une couche de blindage diélectrique (25) agencée en dessous de la couche de câblage,
deux couches de blindage diélectrique agencées au-dessus de la couche de câblage et couvrant toute la zone du patch jetable (2) à l'exception de la zone de l'électrode de blindage sous-jacente (23),
une seconde couche de matériau conducteur (29) agencée au-dessus des couches diélectriques (27, 28) couvrant au moins la zone au-dessus des couches sous-jacentes comprenant les électrodes de détection (21, 22) et l'une ou plusieurs électrodes de blindage (23),
dans lequel l'électrode de blindage (23) est en contact avec la peau/surface d'une personne ou d'un objet et est reliée électriquement à la seconde couche de matériau conducteur (29) via des ouvertures dans les couches diélectriques (27, 28),
le dispositif électronique réutilisable (3) comprenant une carte de circuit imprimé électronique (35) reliée à l'électrode de blindage (23) via un connecteur, fournissant ainsi un blindage électrostatique (45), et
la partie supérieure (31) du patch jetable (2) étant pliable autour de la batterie (13) de sorte que le connecteur (16) soit tourné vers le haut pour une connexion à un second connecteur (34) du dispositif électrique réutilisable (3).

2. Dispositif de capteur selon la revendication 1, dans lequel les électrodes de détection (21, 22) sont des capteurs sans fil comprenant un circuit qui est flottant par rapport à un potentiel de masse.

3. Dispositif de capteur selon la revendication 1 ou 2, dans lequel la distance entre les électrodes de détection (21, 22) est de moins de 10 cm, ou moins de 8, ou moins de 6 cm.

4. Dispositif de capteur selon l'une quelconque des revendications précédentes, dans lequel
le dispositif électronique (3) et les électrodes de détection (21, 22) sont agencés longitudinalement le long de la ligne centrale (*cl*), et dans lequel le dispositif électronique (3) est fixé au patch (2) dans une première extrémité, et les électrodes de détection (21, 22) à différentes positions le long d'une ligne centrale (*cl*) vers l'autre extrémité du patch (2), et
le câblage (18, 19) reliant chacune des électrodes de détection (21, 22) au connecteur de dispositif électronique (16) sont gardés sensiblement égaux en longueur et à proximité immédiate l'un de l'autre dans une conception sensiblement symétrique, réduisant ainsi la zone d'encombrement et la vulnérabilité à la détection de bruit de signa.

5. Dispositif de capteur selon l'une quelconque des revendications précédentes, dans lequel le dispositif électronique comprend :
- un module d'amplification de signal relié aux électrodes de détection (21, 22) sur son côté d'entrée,
- un module de numérisation A/N relié au module d'amplification de signal sur son côté d'entrée,
- un module de stockage module de stockage pour stocker des données échantillonnées étant relié au module de numérisation A/N sur son côté d'entrée,
- un module de communication sans fil pour communiquer des données vers et depuis une ressource informatique distante, et
- un analyseur de signal pour analyser les données échantillonnées étant relié au module de numérisation A/N et ou au module de stockage sur son côté d'entrée, et
- un signal d'ajustement de gain de retour étant sorti du module de stockage ou de l'analyseur de signal et introduit dans le module de numérisation A/N.

6. Dispositif de capteur selon l'une quelconque des revendications précédentes, comprenant en outre :
une électrode de détection de bruit (103) située entre les couches de blindage (27, 28) au-dessus de la couche de câblage, et positionnée longitudinalement entre les électrodes de détection (21, 22),
l'électrode de détection de bruit (103) est reliée par un câblage au module de numérisation en anti-phase par rapport au signal ECG mesuré afin de fournir un mécanisme d'annulation de bruit.

7. Dispositif de capteur selon l'une quelconque des revendications précédentes, dans lequel le couvercle (14) ayant un matériau semi-conducteur (45) pulvérisé à l'intérieur.

8. Dispositif de capteur selon l'une quelconque des revendications précédentes, dans lequel l'électrode de blindage (23) est agencée sur le patch (2) pour être agencée sous la position du dispositif électronique (3).

9. Dispositif de capteur selon l'une quelconque des revendications précédentes, dans lequel le dispositif électronique (3) comprend en outre :
un module de détecteur d'analyseur d'arythmie pour des analyses d'arythmie, et un module d'estimateur d'indice de gravité, dans lequel la sortie provenant du détecteur d'analyseur d'arythmie est utilisée pour une estimation de gravité selon une table d'indices de gravité prédéfinie.

10. Dispositif de capteur selon l'une quelconque des revendications précédentes, dans lequel le dispositif électronique (3) comprend en outre :
un détecteur de déséquilibre d'impédance fournissant une impédance variable pour atténuer le déséquilibre d'impédance sur l'entrée de l'amplificateur de signal, dans lequel des résistances variables 101,102 est fournies entre les électrodes de détection et l'amplificateur de signal, et dans lequel l'impédance variable est fournie par les résistances variables 101,102 étant commandées par logiciel.

11. Dispositif de capteur selon la revendication 10, comprenant en outre une ou plusieurs électrodes de détection de bruit (103) agencées entre les couches d'encre diélectrique (27, 28) pour fournir un signal d'entrée pour des modules d'annulation de bruit, et un câblage reliant les électrodes de détection de bruit (103) au connecteur (16).

12. Dispositif de capteur selon l'une quelconque des revendications précédentes, dans lequel le dispositif électronique (3) comprend en outre un module d'IA entraîné et configuré pour :
- détecter des épisodes d'arythmie dans le signal ECG détecté, et/ou
- détecter et différencier entre une arythmie et un bruit de signal causé par des influences externes ou des variances naturelles d'activités de hart liées à des variances physiques ou émotionnelles, et
- le module d'IA est en outre configuré pour fournir une entrée à un module d'atténuation de bruit fournissant un signal d'annulation de bruit sur l'entrée de l'amplificateur de signal.

13. Procédé de mesure de biopotentiel sans fil en utilisant un dispositif de capteur d'électrocardiogramme, ECG, (1) selon l'une quelconque des revendications 1 à 12, comprenant :
- l'agencement et la fixation du patch de pansement sur le sternum avec la ligne centrale longitudinale alignée avec le sternum, et avec le dispositif électronique (3) dans la position la plus haute,
- la fixation du dispositif électronique (3),
- l'activation d'une routine de mesure,
- le dispositif électronique (3) recevant des lectures de biopotentiel provenant des électrodes de détection (21, 22), et transmettant les données de capteur sans fil à un dispositif informatique (41) et éventuellement à un serveur dorsal (42).

14. Procédé de mesure de biopotentiel sans fil selon la revendication 13, dans lequel l'analyse de données de dispositif de capteur utilisant un système d'IA entraîné basé sur des modèles de réseau neuronal, et
dans lequel le système d'IA entraîné basé sur des modèles de réseau neuronal réside dans l'un quelconque parmi le dispositif électronique (3), le dispositif informatique (41), ou le serveur dorsal (42).

15. Système de mesure de biopotentiel sans fil, le système comprenant :
- un dispositif de capteur d'électrocardiogramme, ECG, (1) selon l'une quelconque des revendications 1 à 12,
- un dispositif informatique (41) étant en communication avec le dispositif de capteur (1) sur une ligne de communication sans fil, et
- un programme d'analyse mis en œuvre par ordinateur, et
le système comprenant en outre un serveur dorsal (42) étant en communication avec le dispositif informatique (41) sur une ligne de communication sans fil, dans lequel le serveur dorsal fournit un ou plusieurs des services suivants pour le dispositif informatique (41) et le dispositif de capteur (1) :
- le téléchargement de données de dispositif de capteur,
- l'analyse de données de dispositif de capteur,
- l'analyse de données de dispositif de capteur en utilisant un système d'IA entraîné basé sur des modèles de réseau neuronal,
- la fourniture d'un résultat pour un affichage sur le dispositif informatique (41),
- la communication de résultats au dispositif informatique (41), et
- la communication de résultats à l'un parmi le WEB de l'utilisateur ou le WEB du docteur.
